(19) Europäisches Patentamt
European Patent Office
Office européen des brevets

(11) Publication number: **0 172 963**
**A1**

(12) **EUROPEAN PATENT APPLICATION**

(21) Application number: **84305960.1**

(22) Date of filing: **30.08.84**

(51) Int. Cl.[4]: **C 07 C 177/00**, C 08 B 37/16, A 61 K 31/557

(43) Date of publication of application: **05.03.86 Bulletin 86/10**

(84) Designated Contracting States: **AT BE CH DE FR GB IT LI LU NL SE**

(71) Applicant: **ONO PHARMACEUTICAL CO., LTD., No. 14, Doshomachi 2-chome Higashi-ku, Osaka-shi Osaka (JP)**

(72) Inventor: **Arai, Yoshinobu, 1-5-8-505, Wakayama-dai Shimamoto-cho, Mishima-gun Osaka (JP)**
Inventor: **Wakatsuka, Hiroshisa, 3-3-708, Miyano-cho, Takatsuki-shi Osaka (JP)**
Inventor: **Sasaki, Yutaro, 11-88-504, Okayamate-cho, Hirakato-shi Osaka (JP)**

(74) Representative: **Bentham, Stephen et al, J.A. Kemp & Co. 14 South Square Gray's Inn, London WC1R 5EU (GB)**

(54) **Prostaglandin analogues, processes for their preparation and compositions containing them.**

(57) Prostaglandin D analogues of the formula:

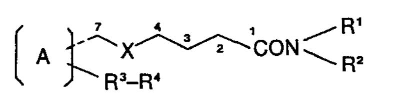

(I)

wherein [A] represents a group of the formula:

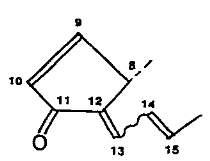

,

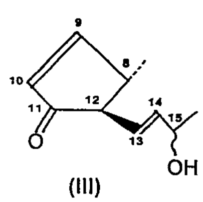

(II) (III)

or

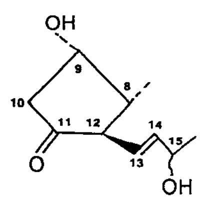

(IV)

wherein X represents ethylene or cis-vinylene, $R^1$ represents a hydrogen or alkyl of 1 to 4 carbon atoms, $R^2$ represents alkyl of 2 to 6 carbon atoms substituted by 1 to 3 hydroxy groups, $R^3$ represents a single bond or alkylene of 1 to 5 carbons atoms, $R^4$ represents alkyl of 1 to 8 carbon atoms unsubstituted or substituted by at least one hydroxy group, cycloalkyl of 4 to 7 carbon atoms unsubstituted or substituted by at least one alkyl of 1 to 8 carbon atoms, or phenyl or phenoxy unsubstituted or substituted by halogen, trifluoromethyl or alkyl of 1 to 4 carbon atoms, with the proviso that when $R^3$ represents a single bond, $R^4$ does not represent unsubstituted or substituted phenoxy, and cyclodextrin clathrates thereof possess anti-tumour activity, increase the number of white blood cells in vivo and synchronize the cell cycle of tumour cell.

EP 0 172 963 A1

DESCRIPTION

PROSTAGLANDIN ANALOGUES, PROCESSES FOR THEIR PREPARATION AND COMPOSITIONS CONTAINING THEM.

This invention relates to new prostaglandin D analogues, processes for their preparation and pharmaceutical compositions containing them.

Prostaglandins are derivatives of prostanoic acid having the following structure:

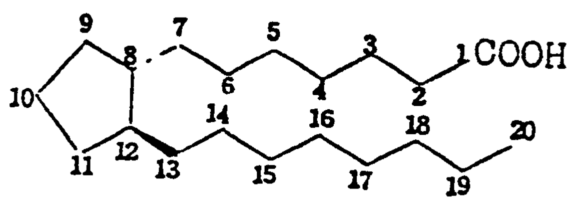

Various types of prostaglandins are known, and their types depend on the structure of the alicyclic ring and the substituents. For example, the alicyclic rings of prostaglandins F (PGF), E (PGE) and D (PGD) have the following structures, respectively:

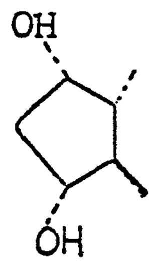 , 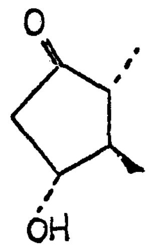 and 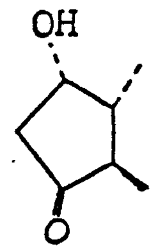

(PGF) (PGE) (PGD)

In the above structural formulae or in the other structural formulae in this specification, according to the generally accepted nomenclature, the dotted line indicates that the substituent attached thereto is behind the ring plane, i.e. is of the α-configuration, the bold line ━ indicates that the substituent attached thereto is in front of the ring plane, i.e. is of the β-configuration, and the wavy line ~ indicates that the substituent attached thereto is of the α-configuration or the β-configuration or a mixture thereof.

These compounds are sub-classified according to the positions of the double bonds in the side chains attached to the alicyclic ring at the 8-position and the 12-position. The PG-1 compound has a <u>trans</u>-double bond between $C_{13}$-$C_{14}$ (<u>trans</u>-$\Delta^{13}$) and PG-2 compound has a <u>cis</u>-double bond between $C_5$-$C_6$ and a trans-double bond between $C_{13}$-$C_{14}$ (<u>cis</u>-$\Delta^5$, <u>trans</u>-$\Delta^{13}$). For example, prostaglandin $D_1$ ($PGD_1$) and

prostaglandin $D_2$ ($PGD_2$) may be expressed by the following structural formulae, respectively:

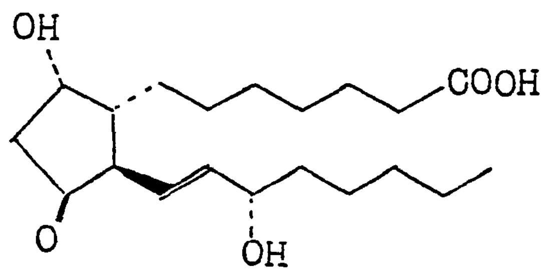

($PGD_1$)

and

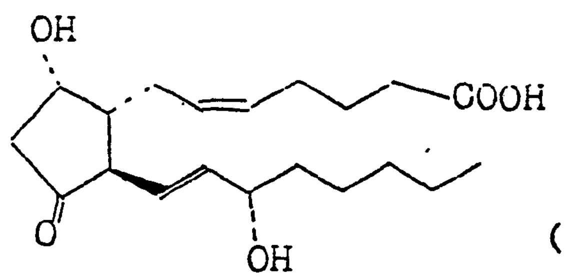

($PGD_2$)

Further, when one or more methylene groups are removed from the aliphatic group attached at the 12-position of the alicyclic ring of a prostaglandin, said compound is called a nor-prostaglandin according to the general rule of the organic nomenclature, and the number of the removed methylene

groups is indicated by adding di-, tri-, etc. before the prefix "nor".

The prostaglandins generally have pharmacological properties. For example, they exert various effects, including the stimulation of contraction of smooth muscles, a hypotensive effect, a diuretic effect, a bronchial dilation effect, the inhibition of lipolysis, the inhibition of platelet aggregation and the inhibition of gastric acid secretion. Therefore, they are useful in treatments of hypertension, thrombosis, asthma and gastric and intestinal ulcers, in the induction of labour and abortion in pregnant mammals, in the prevention of arteriosclerosis and also as diuretics. They are liposoluble substances present in extremely small quantities in the tissues which secrete prostaglandins in vivo in animals.

As a result of research and experimentation to discover novel compounds which have the pharmacological effects of the "natural" prostaglandins, or which have one or more of these properties to an enhanced degree, or which have properties which are not found in the "natural" prostaglandins, it has been discovered that certain novel PGD analogues in which the carboxy group on the 1-position of $PGD_1$ and $PGD_2$ analogues is replaced by a substituted amide group (i.e. a $-CON\begin{smallmatrix}R^1\\R^2\end{smallmatrix}$ group, wherein $R^1$ and $R^2$ are as

hereinafter defined), PGD analogues in which the hydroxy group attached at the 9-position of such compounds is dehydrated to introduce a double bond between $C_9$-$C_{10}$, and PGD analogues in which the hydroxy groups at the 9- and 15-positions of such compounds is dehydrated to introduce double bonds between $C_9$-$C_{10}$, $C_{12}$-$C_{13}$ and $C_{14}$-$C_{15}$ have a surprisingly strong antitumour effect.

Our European Patent Application No. 83303700.5 (Publication No. 98141) describes PGD-amides, 9-deoxy-$\Delta^9$-PGD-amides and 9,15-dideoxy-$\Delta^{9,12,14}$-PGD-amides. The compounds of the present invention are distinguished from those of our earlier application by the presence of a hydroxyalkyl group in the amide grouping: furthermore the compounds of the present invention possess properties not disclosed in our earlier application, namely the ability to increase the number of white blood cells *in vivo* and the ability to synchronise the cell cycle of tumour cells.

The present invention relates to the prostaglandin D analogues of the general formula:

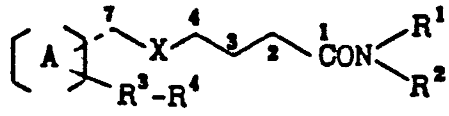

(I)

(wherein the symbol (A) represents a group of the formula:

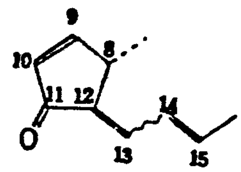

(II)

,

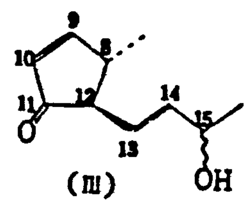

(III)

or

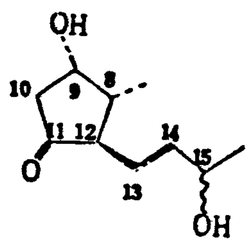

(IV)

(wherein the double bonds between $C_{12}$-$C_{13}$ and $C_{14}$-$C_{15}$ in the formula (II), which may be in the same or different configurations are <u>trans</u>, <u>cis</u> or a mixture thereof,

the double bond between $C_9$-$C_{10}$ is _cis_, the double bonds between $C_9$-$C_{10}$ and $C_{13}$-$C_{14}$ in the formula (III) are, respectively, _cis_ and _trans_ and the double bond between $C_{13}$-$C_{14}$ in the formula (IV) is _trans_, X represents an ethylene group or _cis_-vinylene group, $R^1$ represents a hydrogen atom or a straight-chain or branched-chain alkyl group of 1 to 4 carbon atoms, $R^2$ represents a straight-chain or branched-chain alkyl group of 2 to 6 carbon atoms substituted by 1 to 3 hydroxy groups, $R^3$ represents a single bond or a straight-chain or branched-chain alkylene group of 1 to 5 carbon atoms, $R^4$ represents a straight-chain or branched-chain alkyl group of 1 to 8 carbon atoms unsubstituted or substituted by at least one (e.g. one or two) hydroxy group, a cycloalkyl group of 4 to 7 carbon atoms unsubstituted or substituted by at least one straight-chain or branched-chain alkyl group of 1 to 8 carbon atoms, or a phenyl or phenoxy group unsubstituted or substituted by at least one halogen atom, trifluoromethyl group or straight-chain or branched-chain alkyl group of 1 to 4 carbon atoms, with the proviso that when $R^3$ represents a single bond, $R^4$ does not represent an unsubstituted or substituted phenoxy group 7, and enantiomers thereof and cyclodextrin clathrates thereof.

In the compounds of the general formula (I), there are several asymmetric carbon atoms (at the 8-, 9-, 12- and 15-positions) when /‾A_7 represents the group of the general formula (IV). Similarly, in the general formula (I), the compounds in which /‾A_7 represents the group of the formula (II) or (III) have, respectively, at least one (at the 8-position) or three (at the 8-, 12- and 15-positions) asymmetric carbon atoms.

Furthermore, there is a possibility that other asymmetric centers may occur in groups represented by $R^1$, $R^2$, $R^3$ and $R^4$. The compounds of the general formula (I) of the present invention comprise the isomers including enantiomers, and mixtures thereof, arising from the presence of asymmetric carbon atoms.

In the general formula (I), the alkyl group of 1 to 4 carbon atoms represented by $R^1$ includes methyl, ethyl, propyl and butyl groups and isomers thereof; $R^1$ preferably represents a hydrogen atom, methyl group or ethyl group.

In the general formula (I), the alkyl group of 2 to 6 carbon atoms substituted by 1 to 3 hydroxy groups represented by $R^2$ includes hydroxyethyl, dihydroxyethyl, trihydroxyethyl, hydroxypropyl, dihydroxypropyl, trihydroxypropyl, hydroxybutyl, dihydroxybutyl, trihydroxybutyl, hydroxypentyl, dihydroxypentyl, trihydroxypentyl,

hydroxyhexyl, dihydroxyhexyl, trihydroxyhexyl groups and isomers thereof; $R^2$ preferably represents a 2-hydroxyethyl group, 1,1-bis-(hydroxymethyl)ethyl group (i.e., 1,3-dihydroxy-2-methylpropan-2-yl group), 1-hydroxymethylethyl group (i.e., propanol-2-yl group) or tris(hydroxymethyl)methyl group.

In the general formula (I), the alkylene group of 1 to 5 carbon atoms represented by $R^3$ includes methylene, ethylene, trimethylene, tetramethylene and pentamethylene groups and isomers thereof; $R^3$ preferably represents a single bond or a methylene or ethylene group.

In the general formula (I), the alkyl group of 1 to 8 carbon atoms represented by $R^4$ includes methyl, ethyl, propyl, butyl, pentyl, hexyl, heptyl and octyl groups and isomers thereof; $R^4$ preferably represents a butyl, pentyl or hexyl group either unsubstituted or substituted by one or two methyl or ethyl groups and/or one or two hydroxy groups.

In the general formula (I), the substituted or unsubstituted cycloalkyl group represented by $R^4$ includes cyclobutyl, cyclopentyl, cyclohexyl and cycloheptyl groups, and cyclobutyl, cyclopentyl, cyclohexyl and cycloheptyl groups substituted by one or more methyl, ethyl, propyl, butyl, pentyl, hexyl, heptyl or octyl groups and isomers thereof; $R^4$ preferably represents a cyclobutyl, cyclopentyl or cyclohexyl group either unsubstituted or substituted by one methyl, ethyl, propyl or butyl group.

In the general formula (I), the substituted or unsubstituted phenyl or phenoxy group represented by $R^4$ includes phenyl and phenoxy groups, and phenyl and phenoxy groups substituted by one or more atoms or groups selected from the fluorine and chlorine atoms and trifluoromethyl, methyl, ethyl, propyl or butyl groups; $R^4$ preferably represents a phenyl or phenoxy group either unsubstituted or substituted by one chlorine atom, trifluoromethyl group, methyl or ethyl group.

In the general formula (I), preferred groupings $R^3$-$R^4$ are butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl, 2-ethylheptyl, 4-hydroxybutyl, 5-hydroxypentyl, 6-hydroxyhexyl, cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl, 4-butylcyclohexyl, benzyl, 2-phenylethyl, 4-methylbenzyl, 4-ethylbenzyl, phenoxymethyl, 2-phenoxyethyl, 3-chlorophenoxymethyl, 4-chlorophenoxymethyl, 3-trifluoromethylphenoxymethyl, 4-trifluoromethylphenoxymethyl, 4-methylphenoxymethyl and 4-ethylphenoxymethyl.

Further, in the general formula (I), when [A] represents the formula (III) or (IV), the preferred configuration of the hydroxy group attached to the carbon atom at the 15-position is the α-configuration.

The compounds encompassed by the present invention may be designated as PGD derivatives. For example, the compounds covered by the present invention, and prepared in Example 1 hereinafter, of the formula:

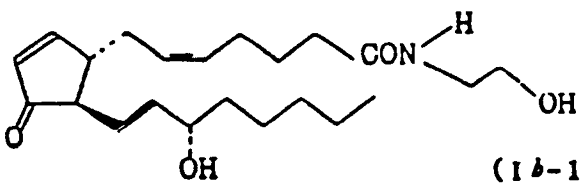

(Ib-1)

prepared in Example 9, of the formula:

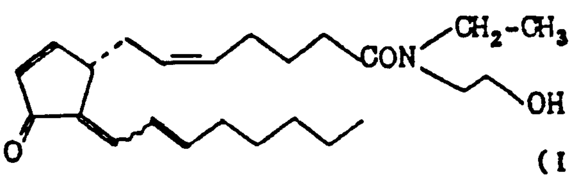

(Ia-1)

prepared in Example 8, of the formula:

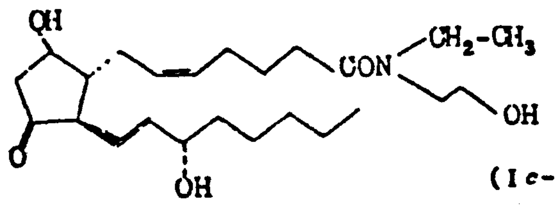

(Ic-1)

can be named, respectively, as 9-deoxy-$\Delta^9$-$PGD_2$ ethanolamide, 9,15-dideoxy-13,14-dyhydro-$\Delta^{9,12,14}$-$PGD_2$ N-ethylethanolamide and $PGD_2$ N-ethylethanolamide. Furthermore, the compounds of the present invention can be named systematically as derivatives of prostanamide of the formula:

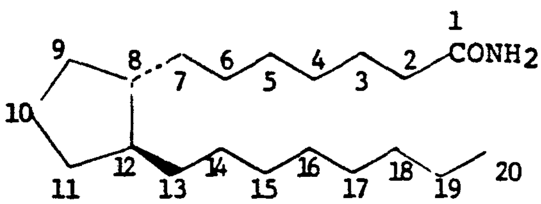

In this case, the compounds of the formula (Ib-1), (Ia-1) and (Ic-1) can be named, respectively, as (5Z, 9Z, 13E)-(15α)-N-(2-hydroxyethyl)-15-hydroxy-11-oxoprosta-5,9,13-trienamide, (5Z, 9Z, 12EZ, 14EZ)-N-ethyl-N-(2-hydroxyethyl)-11-oxoprosta-5,9,12,14-tetraenamide and (5Z, 13E)-N-ethyl-N-(2-hydroxyethyl)-9,15-dihydroxy-11-oxoprosta-5,13-dienamide.

The compounds of the present invention represented by the general formula (I) comprise the compounds of the general formula (Ia), (Ib) and (Ic), namely:

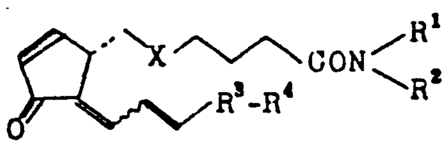

(Ia)

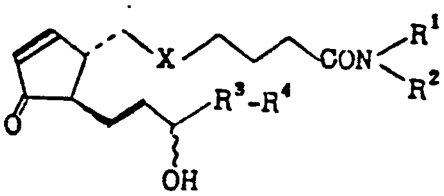

(Ib)

and

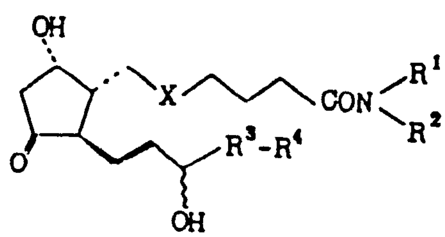

(Ic)

(wherein the various symbols are as hereinbefore defined). These compounds can be prepared by the processes described hereinafter.

According to the present invention, the compounds of the general formulae (Ib) and (Ic) can be prepared, respectively, by hydrolyzing, under acidic conditions, a compound of the general formula:

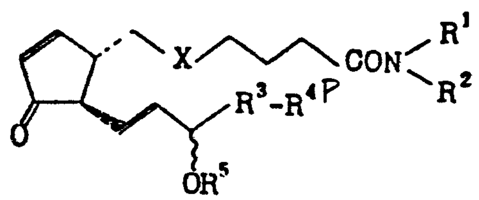

(V)

(wherein $R^5$ represents a 2-tetrahydropyranyl group or 2-tetrahydrofuranyl group either unsubstituted or substituted at least one alkyl group, or a 1-ethoxyethyl group, preferably a 2-tetrahydropyranyl group, $R^{4p}$ represents a group $R^4$ as hereinbefore defined in which, when $R^4$ is a straight-chain or branched-chain alkyl group of 1 to 8 carbon atoms substituted by at least one hydroxy group, the or each hydroxy group is protected by a group $R^5$ as hereinbefore defined and the other symbols are as hereinbefore defined) or a compound of the general formula:

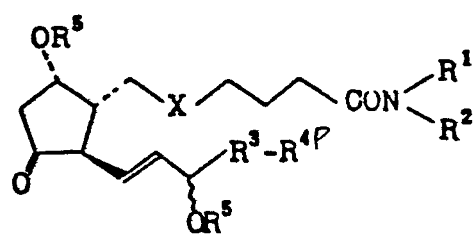

(VI)

(wherein the various symbols are as hereinbefore defined)

This hydrolysis may be conducted:

(1) In an aqueous solution of an organic acid such as acetic acid, propionic acid, oxalic acid or p-toluenesulfonic acid or an aqueous solution of an inorganic acid such as hydrochloric acid, sulfuric acid or phosphoric acid, suitably in the presence of a water-miscible organic solvent, for example, a lower alkanol (preferably methanol) or an ether such as 1,2-dimethoxyethane, dioxan or tetrahydrofuran (preferably tetrahydrofuran) at a temperature of from room temperature to 80°C (preferably at the reflux temperature of the solvent); or,

(2) in an anhydrous lower alkanol such as methanol or ethanol, in the presence of an organic acid such as p-toluenesulfonic acid or trifluoroacetic acid at a temperature of 10 to 45°C.

The hydrolysis is preferably conducted using a mixture of dilute hydrochloric acid and tetrahydrofuran, a mixture of

dilute hydrochloric acid and methanol, a mixture of acetic acid, water and tetrahydrofuran, a mixture of phosphoric acid, water and tetrahydrofuran, or a mixture of p-toluenesulfonic acid and anhydrous methanol.

The compounds of the general formulae (V) and (VI) can be prepared by reacting the acid halide or acid anhydride of the compounds represented by the formulae, respectively:

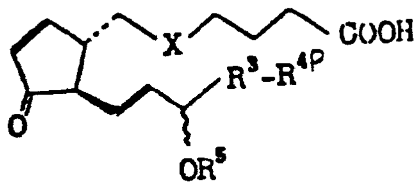
(VII)

(wherein the various symbols are as hereinbefore defined) and

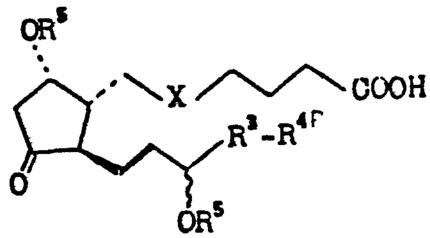
(VIII)

(wherein the various symbols are as hereinbefore defined) with a compound of the general formula:

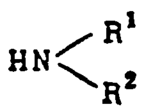

(IX)

(wherein $R^1$ and $R^2$ are as hereinbefore defined), in an inert organic solvent, for example, methylene chloride, tetrahydrofuran, N,N-dimethylformamide, at a temperature of from 0°C to 40°C. This reaction is preferably carried out by reacting a compound of general formula VII or VIII with chloroformic acid, isobutyl chloroformate or trimethylacetyl chloride, in methylene chloride, in the presence of a base such as triethylamine, at room temperature and then reacting the compound thus obtained with a compound of general formula (IX).

According to a further feature of the present invention the compounds of the general formula (Ia) can be prepared by reacting a compound of the general formula:

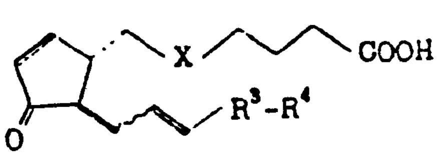

(X)

(wherein the various symbols are as hereinbefore defined) with a compound of the general formula (IX) under the reaction conditions used to prepare the compounds of the general formulae (V) and (VI), respectively, from the compounds of the general formulae (VII) and (VIII).

The compounds of the general formula (VII), (VIII) and (X) can be prepared according to the processes mentioned in the specifications of GB Patent Nos. 1,554,027 or 1,554,028 and United States Patent No. 3,878,239. These compounds can also be produced by the sequence of reaction steps illustrated in Scheme A. In Scheme A, $R^6$ represents an acyl group such as an acetyl or a benzoyl group, $R^7$ represents an alkylsulfonyl group, such as mesyl, or an arylsulfonyl group, such as tosyl, and the other symbols are as hereinbefore defined.

Scheme A

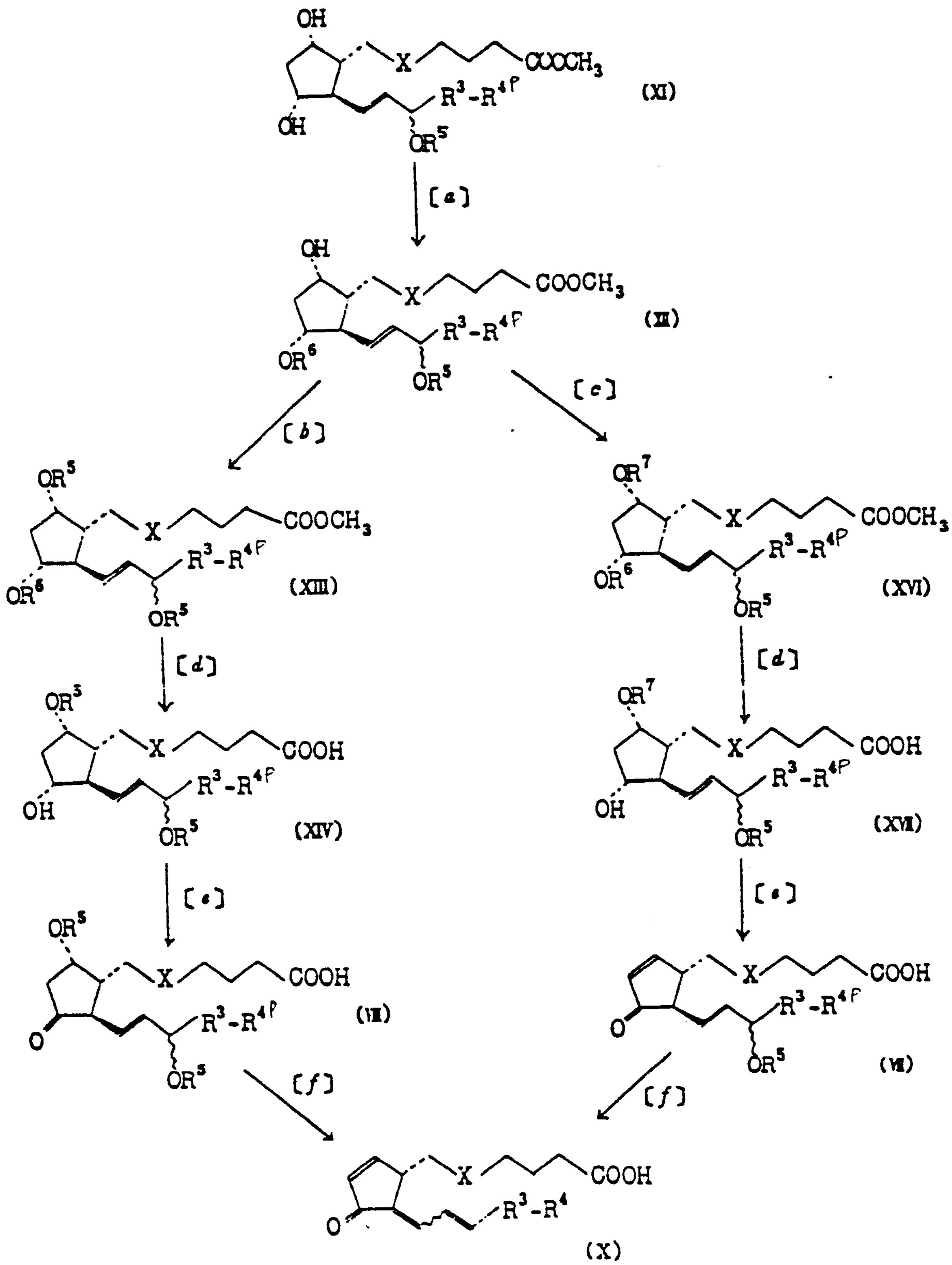

All the steps in Scheme A are carried out by known methods, for example as described below. The step (a) may be conducted by selectively acylating the hydroxy group attached to the 11-position of the compounds of the general formula (XI). Such acylation may be conducted at a temperature not higher than room temperature, preferably at -30°C to -40°C, in an inert organic solvent, such as methylene chloride or in the absence of a solvent, in the presence of a tertiary amine such as pyridine or triethylamine, by using a suitable acyl chloride or acid anhydride, for example, benzoyl chloride.

The step (b) is a reaction to protect a hydroxy group and is conducted using, e.g. 2,3-dihydropyran, 2,3-dihydrofuran, or ethyl vinyl ether, in an inert organic solvent, for example, methylene chloride, chloroform or diethyl ether, in the presence of a condensing agent, for example, p-toluenesulfonic acid, sulfuric acid or trifluoroacetic acid at a temperature of from room temperature to -30°C. Preferably, it is conducted using 2,3-dihydropyran in methylene chloride in the presence of pyridium p-toluenesulfonate or p-toluenesulfonic acid at room temperature.

The step (c) is conducted by reacting with an alkylsulfonyl chloride such as mesyl chloride or an arylsulfonyl chloride such as tosyl chloride at a temperature of

from -30°C to 50°C (i) in an inert organic solvent such as methylene chloride in the presence of a tertiary amine such as pyridine or triethylamine or (ii) in pyridine.

The step (d) is conducted by deacylation at a temperature of from 0°C to 60°C, in an aqueous lower alkanol such as aqueous methanol or aqueous ethanol, using a hydroxide of an alkali metal such as lithium, sodium or potassium.

The step (e) is a reaction to convert the hydroxy group into an oxo group and, in addition to eliminate the $OR^7$ group to form a double bond between $C_9$-$C_{10}$. Such an oxidation reaction is well known, and is described in detail in, for example,

(a) "Synthetic Organic Chemistry III, Organic Synthesis 1", pp. 176-206 (compiled by Tetsuji Kameya and published by Nankodo (Japan) on Aug. 1, 1976), or,

(b) "Compendium of Organic Synthetic Methods", vol. 1, vol. 2, and vol. 3, section 48 or 168 (published by John Wiley & Sons, Inc. (USA) in 1971, 1974, and 1977, respectively).

The oxidation is preferably carried out under mild neutral conditions using, for example, dimethyl-sulphide-N-chlorosuccinimide complex, thioanisole-N-chlorosuccinimide complex,dimethylsulphide-chlorine complex, thioanisole-

chlorine complex (see J. Am. Chem. Soc., 94, 7586 (1972) with respect to these complexes), dicyclohexylcarbodiimide-dimethylsulphoxide complex (see J. Am. Chem. Soc., 87, 5661 (1965)), pyridinium chlorochromate ($C_5H_5NHCrO_3Cl$) (see Tetrahedron Letters, 2647 (1975)), sulphuric anhydride-pyridine complex (see J. Am. Chem. Soc., 89, 5505 (1967)), chromyl chloride (see. J. Am. Chem. Soc., 97, 5929 (1975)), chromium trioxide-pyridine complex (for example, Collins' reagent), Jones' reagent or chromic acid solution (prepared from chromium trioxide, manganese sulfate, sulfuric acid and water), or oxalyl chloride and dimethylsulfoxide (i.e. Swern oxidation); suitably the Collins oxidation, Jones oxidation or Swern oxidation may be employed. The Collins oxidation may be conducted in a halogenated hydrocarbon such as chloroform, methylene chloride or carbon tetrachloride at a temperature of from room temperature to 0°C. The Jones oxidation is generally conducted at a temperature of not higher than room temperature. The Swern oxidation may be conducted by reaction in a halogenated hydrocarbon such as chloroform or methylene chloride at a temperature of from -50°C to -60°C, and then treatment with triethylamine.

The step (f) is a reaction to eliminate $OR^5$ groups and to form a double bond at the same time, which is conducted in an inert organic solvent, for example, methylene chloride, chloroform,

carbon tetrachloride, diethyl ether, N,N-dimethylformamide, tetrahydrofuran or a mixture of two or more such solvents, using an aqueous acid solution, for example, an aqueous solution of an inorganic acid such as hydrochloric acid, hydrobromic acid, sulfuric acid or phosphoric acid or an organic acid such as acetic acid, propionic acid or oxalic acid, at the reflux temperature of the reaction mixture. The reaction is preferably carried out in tetrahydrofuran using 1 N hydrochloric acid at the reflux temperature of the reaction mixture.

The compounds of the general formula (XI) are known compounds and disclosed as chemical products represented by the general formula (XXIII) mentioned in the specification of European Patent Publication No. 98141.

According to a further feature of the invention the compounds of the general formula (Ia) can be prepared by submitting the compounds of the general formula (Ib), (Ic), (V) or (VI) to the reaction of the step (f) illustrated in Scheme A shown above.

As described hereinbefore, the present invention includes both compounds of the natural configuration shown in general formula (I), and isomers, including enantiomers, and mixtures thereof.

The enantiomers may be prepared by the same procedure used to prepare the compounds of natural configuration. The enantiomers of the compounds of the general formula (I) may be prepared from compounds of the following formula (XI'), by the same procedure as described hereinbefore.

In the following Scheme B, an example of the process for the preparation of the enantiomers of the compounds of the general formula (XI) wherein X represents cis-vinylene group is shown. The reaction steps in Scheme B can all be carried out by known methods. The symbols $R^3$ and $R^{4p}$ are as hereinbefore defined.

## Scheme B

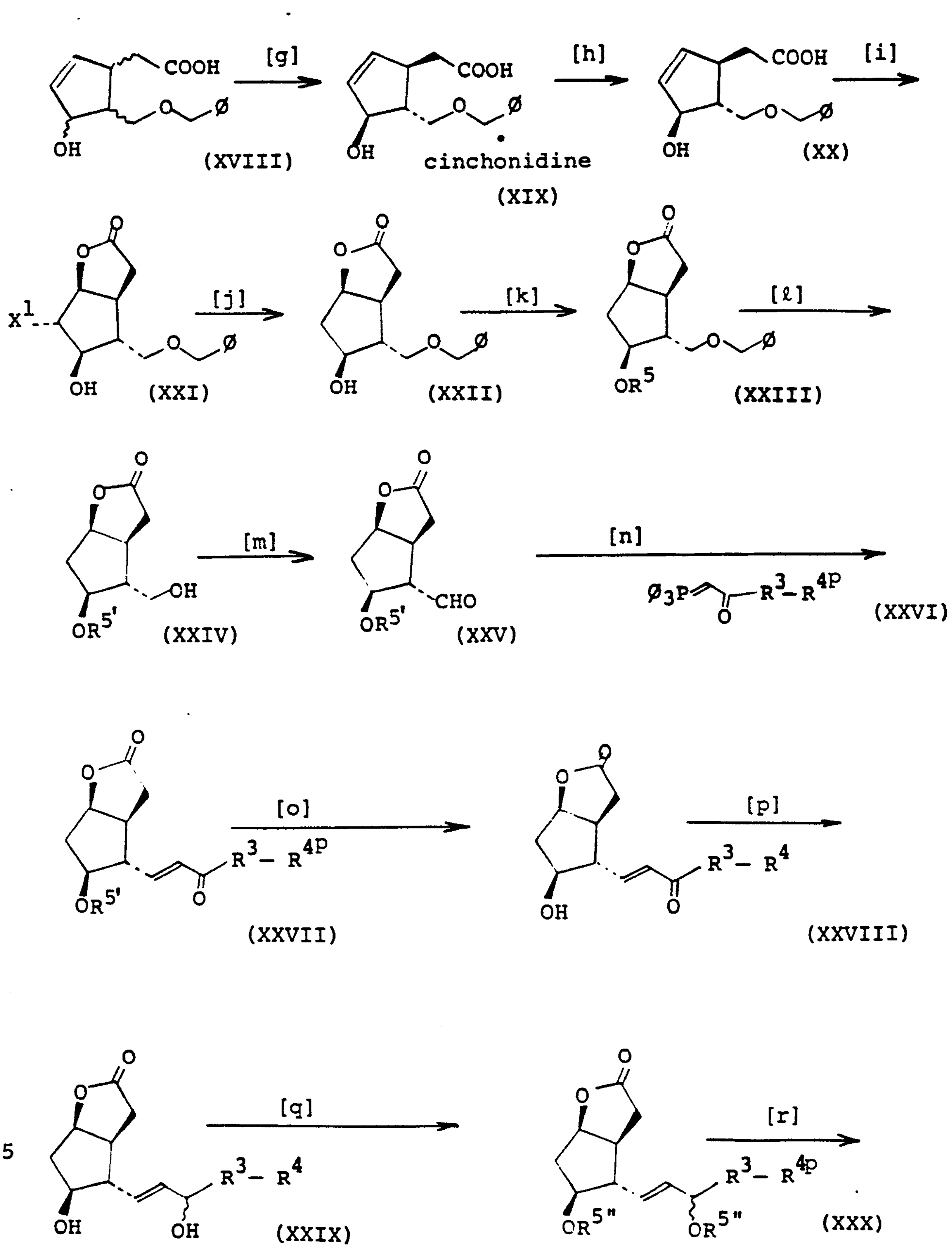


(XVIII) (XIX) (XX) (XXI) (XXII) (XXIII) (XXIV) (XXV) (XXVI) (XXVII) (XXVIII) (XXIX) (XXX)

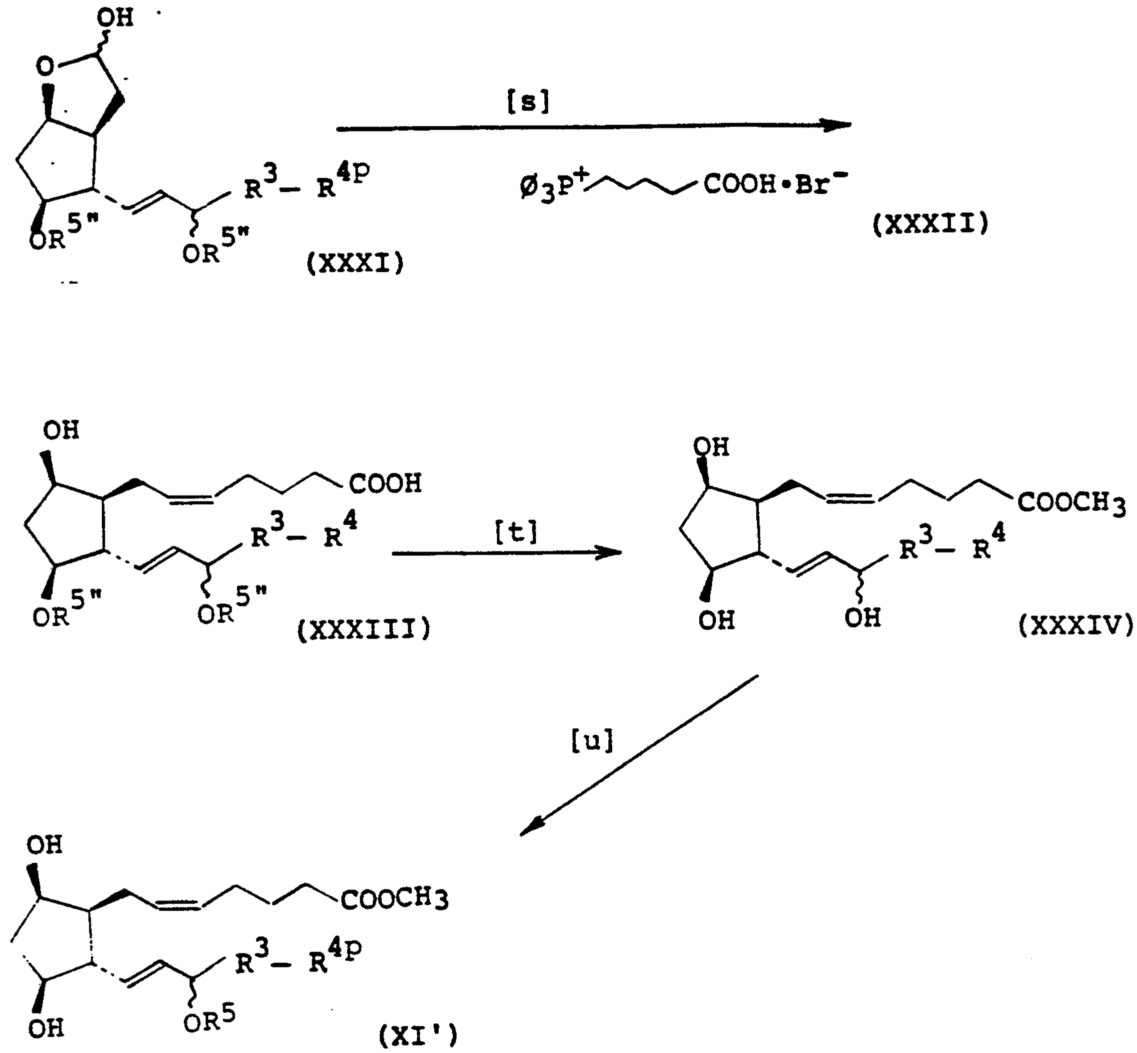


(XXXI) (XXXII) (XXXIII) (XXXIV) (XI')

Reaction steps in Scheme B

[g]&[h] : optical resolution with cinchonidine

[i] : hydrohalogenation and cyclization

[j] : dehalogenation

[k] : protection of hydroxy group

[ℓ] : debenzylation

[m] : oxidation

[n] : Wittig reaction

[o] : removal of protecting group

[p] : reduction

[q] : protection of hydroxy groups

[r] : reduction

[s] : Wittig reaction

[t] : removal of protecting groups of hydroxy groups and esterification

[u] : protection of hydroxy group

Symbols in Scheme B

$X^1$ : halogen atom.

$R^{5'}$, $R^{5''}$ : 2-tetrahydropyranyl or 2-tetrahydrofuranyl group unsubstituted or substituted by at least one of alkyl group or 1-ethoxyethyl group, respectively.

Starting material

Starting material of the formula (XVIII) may be prepared by the procedure described in J. Am. Chem. Soc., 98, 1490 (1971).

The compounds of the present invention may also be prepared by the following procedures from the corresponding prostaglandin D analogues:

Scheme C

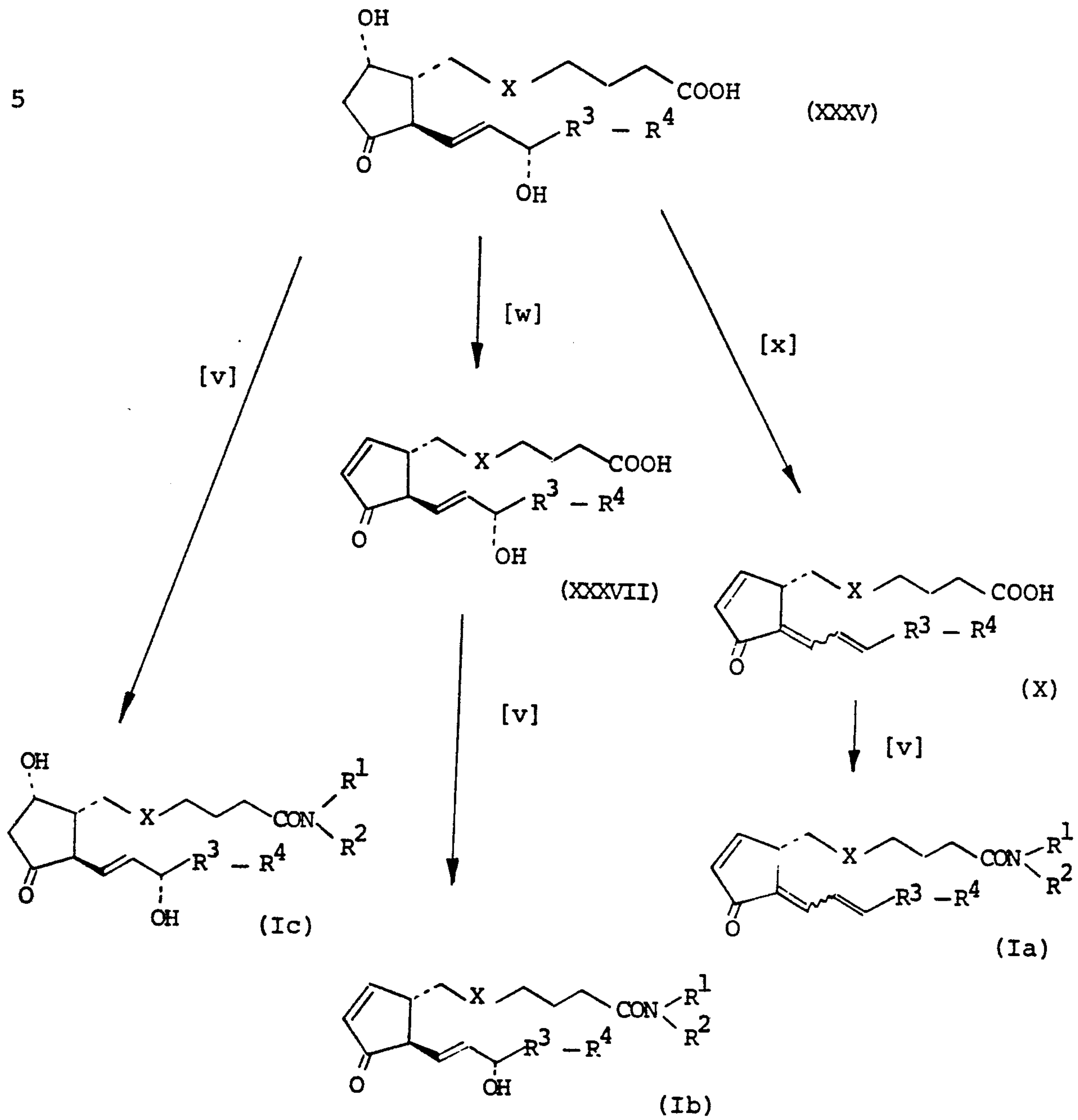


(XXXV)

(XXXVII)

(X)

(Ic)

(Ia)

(Ib)

The reaction steps in Scheme C may all be carried out by known methods. Step (v) is carried out under the conditions hereinbefore described. Step (w) can be carried out under mild dehydration conditions, for example by treatment with a tris hydrochloric acid buffer. Step (x), a dehydration can be carried out by refluxing with an acid.

The cyclodextrin clathrates of the prostaglandin D analogues of the general formula (I) can be prepared using α-, β- or γ-cyclodextrin or mixture thereof, using the procedures described in the specifications of British Patent Nos. 1,351,238 and 1,419,221.
Conversion into the cyclodextrin clathrate serves to increase the stability of the prostaglandin D analogue of the general formula (I).

The prostaglandin D analogues of the general formula (I) and their cyclodextrin clathrates have a specific strong anti-tumour effect, and their toxicity is extremely low. They may therefore be employed as very effective anti-tumour agents in the prevention or therapy of leukemia and solid cancer, and in treatment to produce remission thereof.

In addition, prostaglandin D analogues of the general formula (I) have the effect of increasing the number of white blood cells _in vivo_. Therefore compounds of the present invention are effective as a preventive and therapeutic agent against infection by such microorganisms as bacteria and viruses. Compounds of the present invention may also be used to treat leukocyto-penia induced by administration of conventional anti-tumour agents, because such anti-tumour agents generally cause a decrease in the number of white blood cells as a side effect.

Furthermore, prostaglandin D analogues of the general formula (I) have a synchronization effect on the cell cycle of tumour cells. Cells differentiate and proliferate by repeating such cycle as $G_1$ stage (preparative stage for the synthesis of DNA) $\longrightarrow$ S stage (stage for the synthesis of DNA) $\longrightarrow$ $G_2$ stage (stage for the synthesis of protein) $\longrightarrow$ M stage (stage for cell division) $\longrightarrow$ $G_1$ stage. The compounds of the present invention have the effect of synchronizing tumour cells which exist in four different stages _in vivo_, to the $G_1$ stage. Therefore, when administering a conventional anti-tumour agent which can only attack tumour cells at a particular stage, the compounds of the present invention increase the effect of the said anti-tumour agent,

In an in vitro test on the inhibition of proliferation of cells originated from human mouth tumour (KB-cells) the compounds of the present invention showed an excellent anti-tumour effect. The experimental methods and the results are described below.

Test on Inhibition of Proliferation Using Cells Originated from Human Mouth Tumour (KB-cells)

(Experimental Method)

The cells originated from human mouth tumour (KB-cells) were added to Eagle's MEM culture solution containing 10% bovine fetal serum, the number of cells in the culture solution was adjusted to $1 \times 10^5$ cells/ml, a solution in ethanol of compounds of the present invention was added to give a concentration of 5 μg/ml or below (3 points) and the mixture was subjected to stationary culture at 37°C for 4 days. As a control, a culture solution containing 0.1% of ethanol was similarly cultured. After the culture, the cells were stained by the Trypan Blue staining method, and the surviving cell number was measured to determine the $IC_{50}$ value from the degree of inhibition relative to the control. The results are given below.

Inhibition of Proliferation Using Cells Originated from Human Mouth Tumor (KB-Cells)

| Test Compound | Inhibition of Proliferation ($IC_{50}$, μg/ml) |
|---|---|
| 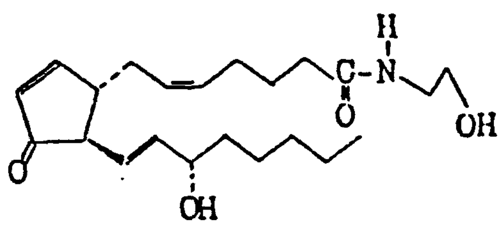 | 1. 8 |
| 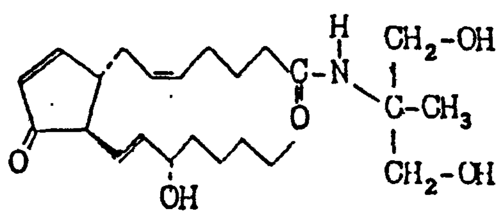 | 2. 1 |
| 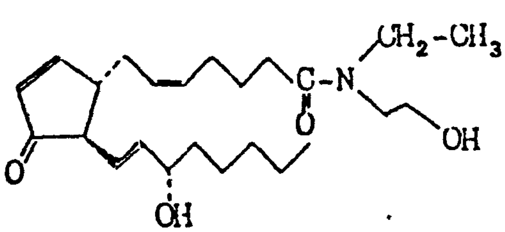 | 1. 6 |

Of the compounds represented by general formula (Ia) which fall within the present invention, specific examples of preferred compounds include the corresponding ethanolamides, N-ethylethanolamides, N-methylethanolamides, 2-methylpropane-1,3-diol-2-amides, tris(hydroxymethyl)methylamides and propanol-2-amides of:

9,15-Dideoxy-13,14-dihydro-$\Delta^{9,12,14}$-$PGD_1$

9,15-Dideoxy-13,14-dihydro-$\Delta^{9,12,14}$-$PGD_2$

9,15-dideoxy-13,14-dihydro-20-hydroxy-$\Delta^{9,12,14}$-$PGD_1$

9,15-dideoxy-13,14-dihydro-20-hydroxy-$\Delta^{9,12,14}$-$PGD_2$

9,15-Dideoxy-13,14-dihydro-16-methyl-$\Delta^{9,12,14}$-$PGD_1$

9,15-Dideoxy-13,14-dihydro-16-methyl-$\Delta^{9,12,14}$-$PGD_2$

9,15-Dideoxy-13,14-dihydro-16,16-dimethyl-$\Delta^{9,12,14}$-$PGD_1$

9,15-Dideoxy-13,14-dihydro-16,16-dimethyl-$\Delta^{9,12,14}$-$PGD_2$

9,15-Dideoxy-13,14-dihydro-17,20-dimethyl-$\Delta^{9,12,14}$-$PGD_1$

9,15-Dideoxy-13,14-dihydro-17,20-dimethyl-$\Delta^{9,12,14}$-$PGD_2$

9,15-Dideoxy-13,14-dihydro-15-(1-butylcyclobutyl)-16,17,18,19,20-pentanor-$\Delta^{9,12,14}$-$PGD_1$

9,15-Dideoxy-13,14-dihydro-15-(1-butylcyclobutyl)-16,17,18,19,20-pentanor-$\Delta^{9,12,14}$-$PGD_2$

9,15-Dideoxy-13,14-dihydro-15-cyclopentyl-16,17,18,19,20-pentanor-$\Delta^{9,12,14}$-$PGD_1$

9,15-Dideoxy-13,14-dihydro-15-cyclopentyl-16,17,18,19,20-pentanor-$\Delta^{9,12,14}$-$PGD_2$

9,15-Dideoxy-13,14-dihydro-15-(3-propylcyclopentyl)-16,17,18,19,20-pentanor-$\Delta^{9,12,14}$-$PGD_1$

9,15-Dideoxy-13,14-dihydro-15-(3-propylcyclopentyl)-16,17,18,19,20-pentanor-$\Delta^{9,12,14}$-$PGD_2$

9,15-Dideoxy-13,14-dihydro-15-(3-butylcyclopentyl)-16,17,18,19,20-pentanor-$\Delta^{9,12,14}$-$PGD_1$

9,15-Dideoxy-13,14-dihydro-15-(3-butylcyclopentyl)-16,17,18,19,20-pentanor-$\Delta^{9,12,14}$-$PGD_2$

9,15-Dideoxy-13,14-dihydro-15-(4-butylcyclo~~pentyl~~hexyl)-16,17,18,19,20-pentanor-$\Delta^{9,12,14}$-$PGD_1$

9,15-Dideoxy-13,14-dihydro-15-(4-butylcyclo~~pentyl~~hexyl)-16,17,18,19,20-pentanor-$\Delta^{9,12,14}$-$PGD_2$

9,15-Dideoxy-13,14-dihydro-16-phenyl-17,18,19,20-tetranor-$\Delta^{9,12,14}$-$PGD_1$

9,15-Dideoxy-13,14-dihydro-16-phenyl-17,18,19,20-tetranor-$\Delta^{9,12,14}$-$PGD_2$

9,15-Dideoxy-13,14-dihydro-16-phenoxy-17,18,19,20-tetranor-$\Delta^{9,12,14}$-$PGD_1$

9,15-Dideoxy-13,14-dihydro-16-phenoxy-17,18,19,20-tetranor-$\Delta^{9,12,14}$-$PGD_2$

9,15-Dideoxy-13,14-dihydro-16-(3-chlorophenoxy)-17,18,19,20-tetranor-$\Delta^{9,12,14}$-$PGD_1$

9,15-Dideoxy-13,14-dihydro-16-(3-chlorophenoxy)-17,18,19,20-tetranor-$\Delta^{9,12,14}$-$PGD_2$

9,15-Dideoxy-13,14-dihydro-16-(3-trifluoromethyl-phenoxy)-17,18,19,20-tetranor-$\Delta^{9,12,14}$-$PGD_1$

and,

9,15-Dideoxy-13,14-dihydro-16-(3-trifluoromethyl-phenoxy)-17,18,19,20-tetranor-$\Delta^{9,12,14}$-$PGD_2$

Of the compounds represented by general formula (Ib) which fall within the present invention, specific examples of preferred compounds include the corresponding ethanolamides, N-ethylethanolamides, N-methylethanolamides, 2-methylpropane-1,3-diol-2-amides , tris(hydroxymethyl)methylamides and propanol-2-amides of:

9-Deoxy-$\Delta^{9}$-$PGD_1$

9-Deoxy-$\Delta^9$-$PGD_2$

9-deoxy-20-hydroxy-$\Delta^9$-$PGD_1$

9-deoxy-20-hydroxy-$\Delta^9$-$PGD_2$

9-Deoxy-16-methyl-$\Delta^9$-$PGD_1$

9-Deoxy-16-methyl-$\Delta^9$-$PGD_2$

9-Deoxy-16,16-dimethyl-$\Delta^9$-$PGD_1$

9-Deoxy-16,16-dimethyl-$\Delta^9$-$PGD_2$

9-Deoxy-17,20-dimethyl-$\Delta^9$-$PGD_1$

9-Deoxy-17,20-dimethyl-$\Delta^9$-$PGD_2$

9-Deoxy-15-(1-butylcyclobutyl)-16,17,18,19,20-pentanor-$\Delta^9$-$PGD_1$

9-Deoxy-15-(1-butylcyclobutyl)-16,17,18,19,20-pentanor-$\Delta^9$-$PGD_2$

9-Deoxy-15-cyclopentyl-16,17,18,19,20-pentanor-$\Delta^9$-$PGD_1$

9-Deoxy-15-cyclopentyl-16,17,18,19,20-pentanor-$\Delta^9$-$PGD_2$

9-Deoxy-15-(3-propylcyclopentyl)-16,17,18,19,20-pentanor-$\Delta^9$-$PGD_1$

9-Deoxy-15-(3-propylcyclopentyl)-16,17,18,19,20-pentanor-$\Delta^9$-$PGD_2$

9-Deoxy-15-(3-butylcyclopentyl)-16,17,18,19,20-pentanor-$\Delta^9$-$PGD_1$

9-Deoxy-15-(3-butylcyclopentyl)-16,17,18,19,20-pentanor-$\Delta^9$-$PGD_2$

9-Deoxy-15-(4-butylcyclo~~pent~~hexyl)-16,17,18,19,20-pentanor-$\Delta^9$-$PGD_1$

9-Deoxy-15-(4-butylcyclo~~pent~~hexyl)-16,17,18,19,20-pentanor-$\Delta^9$-$PGD_2$

9-Deoxy-16-phenyl-17,18,19,20-tetranor-$\Delta^9$-$PGD_1$

9-Deoxy-16-phenyl-17,18,19,20-tetranor-$\Delta^9$-$PGD_2$

9-Deoxy-16-phenoxy-17,18,19,20-tetranor-$\Delta^9$-$PGD_1$

9-Deoxy-16-phenoxy-17,18,19,20-tetranor-$\Delta^9$-$PGD_2$

9-Deoxy-16-(3-chlorophenoxy)-17,18,19,20-tetranor-$\Delta^9$ $PGD_1$

9-Deoxy-16-(3-chlorophenoxy)-17,18,19,20-tetranor-$\Delta^9$-$PGD_2$

9-Deoxy-16-(3-trifluoromethylphenoxy)-17,18,19,20-tetranor-$\Delta^9$-$PGD_1$

and,

9-Deoxy-16-(3-trifluoromethylphenoxy)-17,18,19,20-tetranor-$\Delta^9$-$PGD_2$

Of the compounds represented by general formula (Ic) which fall within the present invention, specific examples of preferred compounds include the corresponding ethanolamides, N-ethylethanolamides, N-methylethanolamides, 2-methylpropane-1,3-diol-2-amides , tris(hydroxymethyl)methylamides and propanol-2-amides of:

$PGD_1$

$PGD_2$

20-hydroxy-$PGD_1$

20-hydroxy-$PGD_2$

16-Methyl-$PGD_1$

16-Methyl-$PGD_2$

16,16-Dimethyl-$PGD_1$

16,16-Dimethyl-$PGD_2$

17,20-Dimethyl-$PGD_1$

17,20-Dimethyl-$PGD_2$

15-(1-Butylcyclobutyl)-16,17,18,19,20-pentanor-$PGD_1$

15-(1-Butylcyclobutyl)-16,17,18,19,20-pentanor-$PGD_2$

15-Cyclopentyl-16,17,18,19,20-pentanor-$PGD_1$

15-Cyclopentyl-16,17,18,19,20-pentanor-$PGD_2$

15-(3-Propylcyclopentyl)-16,17,18,19,20-pentanor-$PGD_1$

15-(3-Propylcyclopentyl)-16,17,18,19,20-pentanor-$PGD_2$

15-(3-Butylcyclopentyl)-16,17,18,19,20-pentanor-$PGD_1$

15-(3-Butylcyclopentyl)-16,17,18,19,20-pentanor-$PGD_2$

15-(4-Butylcyclo~~pent~~hexyl)-16,17,18,19,20-pentanor-$PGD_1$

15-(4-Butylcyclo~~pent~~hexyl)-16,17,18,19,20-pentanor-$PGD_2$

16-Phenyl-17,18,19,20-tetranor-$PGD_1$

16-Phenyl-17,18,19,20-tetranor-$PGD_2$

16-Phenoxy-17,18,19,20-tetranor-$PGD_1$

16-Phenoxy-17,18,19,20-tetranor-$PGD_2$

16-(3-Chlorophenoxy)-17,18,19,20-tetranor-$PGD_1$

16-(3-Chlorophenoxy)-17,18,19,20-tetranor-$PGD_2$

16-(3-Trifluoromethylphenoxy)-17,18,19,20-tetranor-$PGD_1$

and,

16-(3-Trifluoromethylphenoxy)-17,18,19,20-tetranor-$PGD_1$

The following Reference Examples and

Examples illustrate the invention. In the Reference Examples and Examples, "m.p.", "TLC", "NMR", "IR" and "Mass" represent "Melting point", "Thin layer chromatography", "Nuclear magnetic resonance spectrum", "Infrared absorption spectrum" and "Mass spectrum", respectively. The ratios of the solvents used in chromatographic separations are by volume. The solvents in parentheses in "TLC" show the developing solvents. Unless otherwise specified, "IR" was measured by the liquid film method and "NMR" was measured in a deuterochloroform ($CDCl_3$) solution.

Reference Example 1

(5Z,13E)-(9α,11α,15α)-11-Benzoyloxy-9-methanesulfonyloxy-15-(2-tetrahydropyranyloxy)prosta-5,13-dienoic acid methyl ester:

To a solution of 2.8 g of (5Z,13E)-(9α,11α,15α)-9-hydroxy-11-benzoyloxy-15-(2-tetrahydropyranyloxy)prosta-5,13-dienoic acid methyl ester (prepared in accordance with the process described in European Patent Publication No. 98141, Reference Example 2) in 50 ml of dry methylene chloride, 1.12 ml of triethylamine and 0.58 ml of mesyl chloride were added at -20°C under an argon atmosphere. The resulting mixture was stirred for 15 minutes at the same temperature. The reaction mixture was poured into 150 ml of

ice water. The mixture was extracted with diethyl ether. The extract was washed in succession with water and a saturated sodium chloride aqueous solution. After drying, the extract was concentrated under reduced pressure to give 3.23 g of the title compound having the following physical characteristics:

TLC (n-hexane : ethyl acetate = 2:1): Rf = 0.51;

NMR: $\delta$ = 8.1-7.7 (2H, m), 7.6-7.1 (3H, m ), 5.7-4.9 (6H, m), 3.7 (3H, s), 3.0 (3H, s).

Reference Example 2

(5Z,13E)-(9α,11α,15α)-11-Hydroxy-9-methanesulfonyloxy-15-(2-tetrahydropyranyloxy)prosta-5,13-dienoic acid:

To a solution of 3.23 g of the 11-benzoyl compound (prepared in Reference Example 1) in 133 ml of methanol, a suspension of 12.6 g of lithium hydroxide monohydrate in 19 ml of water was added at 5°C. The mixture was stirred for 2 hours at the same temperature and for further 5 hours at room temperature. The reaction mixture was poured into 300 ml of ice water and 1 N hydrochloric acid was added to the mixture to adjust pH to 3. Then the mixture was extracted with ethyl acetate. The extract was washed successively with water and a saturated sodium chloride aqueous solution. After drying, the extract was concentrated under reduced pressure. The residue was purified by column chromatography on silica gel

using a solvent mixture of n-hexane and ethyl acetate and then using ethyl acetate, as eluents to give 2.01 g of the title compound having the following physical characteristics:

TLC (chloroform : tetrahydrofuran : acetic acid = 30:6 :1): Rf = 0.44;

NMR: $\delta$ = 6.4-5.6 (2H, m), 5.7-5.2 (4H, m), 5.15-4.9 (1H, m), 4.8-4.6 (1H, m), 4.3-3.1 (4H, m), 3.0 (3H, s);

IR: $\nu$ = 3650-2400, 2930, 2860, 1720, 1340, 1170, 970 $cm^{-1}$

## Reference Example 3

(5Z,9Z,13E)-(15$\alpha$)-11-Oxo-15-(2-tetrahydropyranyloxy)prosta-5,9,13-trienoic acid:

To a solution of 1.24 g of the 9-mesyl compound (prepared in Reference Example 2) in 12 ml of acetone, 4.5 ml of Jones reagent was dropwise added at -30°C. The mixture was stirred for 20 minutes at -20°C. The reaction mixture was diluted with diethyl ether followed by washing successively with water and a saturated sodium chloride aqueous solution. After drying, the residue was concentrated under reduced pressure. The residue was purified by column chromatography on silica gel using a mixture of ethyl acetate and n-hexane as an eluent to give 610 mg of the title compound having the following physical characteristics:

TLC (ethyl acetate: n-hexane

= 2 : 1): Rf = 0.27

NMR: $\delta$=7.64-7.52 (1H, m), 6.22-6.10 (1H, m), 5.7-5.2 (4H, m), 4.8-4.6 (1H, m), 1.0-0.8 (3H, m);

IR: $\nu$ =1715, 1590, 1020 $cm^{-1}$

## Reference Example 4

The following compounds were obtained from compounds described in European Patent Publication No. 98141, Reference Examples 2(b), 2(c), 2(e) and 2(a), respectively, by the same procedures as described in Reference Examples 1, 2 and 3.

(a) (5Z,9Z,13E)-(15α,17α)-17,20-Dimethyl-11-oxo-15-(2-tetrahydropyranyloxy)prosta-5,9,13-trienoic acid:

TLC (ethyl acetate : n-hexane = 2:1): Rf = 0.31;

IR: $\nu$ =2940, 1710, 1590 $cm^{-1}$;

NMR: $\delta$=7.63~7.52 (1H, m), 6.21~6.10 (1H, m), 5.7~5.2 (4H, m), 4.8~4.6 (1H, m), 1.0~0.8 (6H, m).

(b) (5Z,9Z,13E)-(15α)-16,18-Ethano-20-methyl-11-oxo-15-(2-tetrahydropyranyloxy)prosta-5,9,13-trienoic acid:

TLC (ethyl acetate : n-hexane = 2:1): Rf = 0.30;

IR: $\nu$ =1713, 1590 $cm^{-1}$;

NMR: $\delta$=7.64~7.53 (1H, m), 6.23~6.10 (1H, m), 5.73~5.20 (4H, m), 4.8~4.6 (1H, m), 1.0~0.8 (3H, m).

(c) (5Z,9Z,13E)-(15α)-16-(3-Chlorophenoxy)-11-oxo-15-(2-tetrahydropyranyloxy)-17,18,19,20-tetranorprosta-5,9,13-trienoic acid:

TLC (ethyl acetate : n-hexane = 2:1): Rf = 0.27;

IR: ν =1715、1610、1590 $cm^{-1}$;

NMR: δ=8.1~7.8 (2H, m)、7.6~6.7 (5H, m)、6.2~6.1 (1H, m)、5.7~5.2 (4H, m)。

(d) (9Z,13E)-(15α)-11-Oxo-15-(2-tetrahydropyranyloxy)-prosta-9,13-dienoic acid:

TLC (ethyl acetate : n-hexane = 2:1): Rf = 0.29;

IR: ν =2940、1735、1715、1590 $cm^{-1}$;

NMR: δ=7.62~7.50 (1H, m)、6.20~6.10 (1H, m)、5.7~5.4 (2H, m)、4.8~4.6 (1H, m)、1.0~0.8 (3H, m)。

## Reference Example 5

(5Z,13E)-(9α,11α,15α)-9,15-Bis(2-tetrahydropyranyloxy)-11-benzoyloxyprosta-5,13-dienoic acid methyl ester:

To a solution of 3.77 g of (5Z,13E)-(9α,11α,15α)-9-hydroxy-11-benzoyloxy-15-(2-tetrahydropyranyloxy)prosta-5,13-dienoic acid (prepared in accordance with the process described in European Patent Publication No. 98141, Reference Example 2) in 15 ml of methylene chloride, 1 ml of 2,3-dihydropyran was dropwise added at room temperature under an argon atmosphere. Thereafter a catalytic amount of p-toluenesulfonic acid was added to the mixture. After stirring for 15 minutes, 0.1 ml of triethylamine was added. The resulting mixture was concentrated under reduced pressure. The residue was purified by column chromatography on silica gel using a solvent mixture of n-hexane and ethyl acetate as an eluent to give 3.95 g of the title compound having the following physical characteristics:

TLC (n-hexane : ethyl acetate = 2 : 1): Rf = 0.45;

NMR: δ=8.1~7.9 (2H, m)、7.6~7.3 (3H, m)、5.7~5.3 (4H, m)、5.3~5.0 (1H, m)、4.7~4.4 (2H, m)、3.7 (3H, s)、0.8 (3H, t);

IR: ν =2940、1735、1720、1450、1270、1110、1020cm⁻¹。

Reference Example 6

(5Z,13E)-(9α,11α,15α)-9,15-Bis(2-tetrahydropyranyloxy)-11-hydroxyprosta-5,13-dienoic acid:

To a solution of 500 mg of (5Z,13E)-(9α,11α,15α)-9,15-bis(2-tetrahydropyranyloxy)-11-benzoyloxyprosta-5,13-dienoic acid (prepared in Reference Example 5) in 4.8 ml of methanol, 1.6 ml of a 2 N aqueous potassium hydroxide solution was added. After the mixture was stirred at 50°C for 1.5 hour, the mixture was cooled to 5°C and pH was adjusted to 2 by adding 1 N hydrochloric acid thereto. To the reaction mixture, 100 ml of ice water was added and the mixture was extracted with ethyl acetate. The extract was washed in succession with water and a saturated sodium chloride aqueous solution. After the extract was dried over anhydrous magnesium sulfate, the extract was concentrated under reduced pressure to give 483 mg of the title compound as crude product. The obtained product was provided for subsequent reaction without purification.

TLC (benzene : ethyl acetate) = 2 : 1):

Rf = 0.07

Reference Example 7

(5Z,13E)-(9 α,15α)-9,15-Bis(2-tetrahydropyranyloxy)-11-oxo-prosta-5,13-dienoic acid:

A solution of 1.04 g of (5Z,13E)-(9α,11α,15α)-9,15-bis-(2-tetrahydropyranyloxy)-11-hydroxyprosta-5,13-dienoic acid

(prepared in Reference Example 6) in 10 ml of acetone was cooled to -30°C and Jones reagent (prepared from 378 mg of chromium trioxide, 0.331 ml of conc. sulfuric acid and 1.41 ml of water) was dropwise added to the solution. After stirring for 30 minutes at the same temperature, 1 ml of isopropyl alcohol and then 100 ml of water were added thereto. The reaction mixture was extracted 3 times with 50 ml (150 ml in total) of diethyl ether. The extract was successively washed with water and a saturated sodium chloride aqueous solution. After the extract was dried over anhydrous magnesium sulfate, it was concentrated under reduced pressure. The residue was purified by column chromatography on silica gel using a solvent mixture of cyclohexane and ethyl acetate (2 : 1) as an eluent to give 652 mg of the title compound having the following physical characteristics:

TLC (benzene : ethyl acetate = 1 : 2):

Rf = 0.7;

NMR: $\delta$=10.1 (1H, s), 5.7~5.2 (4H, m),

4.8~4.5 (2H, m), 1.0~0.7 (3H, m);

Mass: m/e=436, 418.

## Reference Example 8

(5Z,9Z,12EZ,14EZ)-11-Oxoprosta-5,9,12,14-tetraenoic acid:

A mixture of 11 mg of $PGD_2$ (prepared in accordance with the process described in United States Patent No. 3,878,239 Example 1 (B)), 0.28 ml of 1N hydrochloric acid and 0.28 ml of tetrahydrofuran was refluxed for 20 minutes. Thereafter, 50 ml of diethyl ether was added to the mixture. The resulting mixture was washed with a saturated sodium chloride aqueous solution. After drying over anhydrous magnesium sulfate, the mixture was concentrated under reduced pressure. The residue was purified by column chromatography on silica gel using a solvent mixture of n-hexane and ethyl acetate (2 : 1) as an eluent to give 3 mg of the title compound having the following physical characteristics:

TLC (chloroform : tetrahydrofuran : acetic acid = 30 : 6 : 1): Rf = 0.62;

NMR: $\delta$=7.47(1H, dd), 6.96(1H, d), 6.46~6.15(3H, m), 5.55~5.28(2H, m), 3.65~3.52(1H, m), 0.90(3H, t);

IR: $\nu$ =3600~2400, 2920, 1725, 1700, 1625 $cm^{-1}$.

Mass: m/e=316.

Reference Example 9

(5Z,9Z,13E)-(15α)-N-(2-Hydroxyethyl)-15-(2-tetrahydropyranyloxy)-11-oxoprosta-5,9,13-trienamide:

To a solution of 200 mg of (5Z,9Z,13E)-(15α)-15-(2-tetrahydropyranyloxy)-11-oxoprosta-5,9,13-trienoic acid (prepared in Reference Example 3) in 10 ml of methylene chloride, 0.1 ml of triethylamine was added at room temperature under an argon atmosphere. After 80 µl of isobutyl chloroformate was dropwise added to the mixture, the resulting mixture was stirred for 15 minutes. To the solution, 40 µl of ethanolamine was added. After stirring for 15 minutes, the mixture was diluted with methylene chloride, washed with an aqueous sodium bicarbonate solution and a saturated sodium chloride aqueous solution, dried over anhydrous magnesium sulfate and then concentrated under reduced pressure. The residue was purified by column chromatography on silica gel using a solvent mixture of ethyl acetate and n-hexane as an eluent to give 175 mg of the title compound having the following physical characteristics. Yield, 79%.

TLC (chloroform : tetrahydrofuran : acetic acid = 10 : 2 : 1): Rf = 0.36;

IR: $\nu$ =3320, 1705, 1650, 1540, 1240, 1075, 1020, 980 $cm^{-1}$;

NMR: $\delta$=7.6 (1H, dd), 6.16 (1H, dd), 5.2-5.8 (4H, m), 4.6-4.8 (1H, m), 3.0-4.2 (7H, m), 2.73-3.0 (1H, m), 2.6-2.73 (1H, m), 0.7-1.0 (3H, m).

## Reference Example 10

(5Z,9Z,13E)-(15α)-N-(1,3-Dihydroxy-2-methylpropan-2-yl)-15-(2-tetrahydropyranyloxy)-11-oxoprosta-5,9,13-trienamide:

To a solution of 250 mg of (5Z,9Z,13E)-(15α)-15-(2-tetrahydropyranyloxy)-11-oxoprosta-5,9,13-trienoic acid (prepared in Reference Example 3) in 10 ml of methylene chloride, 125 μl of triethylamine was added at room temperature under an argon atmosphere. After 100 μl of isobutyl chloroformate was dropwise added to the mixture, the resulting mixture was stirred for 15 minutes. To the solution, 95 mg of 2-amino-2-methylpropane-1,3-diol was added and stirred for 2 hours at the same temperature. The solution was diluted with methylene chloride, washed with an aqueous sodium bicarbonate solution and a sodium chloride aqueous solution, dried over anhydrous magnesium sulfate and then concentrated under reduced pressure. The residue was purified by column chromatography on silica gel using a solvent mixture of ethyl acetate and n-hexane as an eluent to give 190 mg of the title compound having the following physical characteristics. Yield, 63%.

TLC (chloroform : tetrahydrofuran : acetic acid = 10 : 2 : 1): Rf = 0.34;

IR: $\nu$ = 3350, 1705, 1645, 1540, 1450, 1240, 980 $cm^{-1}$;

NMR: $\delta$ = 7.57 (1H, dd), 6.1 - 6.2 (1H, m), 5.9~6.1 (1H, m), 5.3~5.87 (4H, m), 4.6~4.75 (1H, m), 3.4~4.2 (7H, m), 2.75~2.93 (1H, m), 2.6~2.72 (1H, m) 1.22 (3H, s), 0.8~1.0 (3H, m);

Mass: m/e = 505 ($M^+$), 487, 474, 456, 421 403, 372, 321.

## Reference Example 11

(5Z,9Z,13E)-(15α)-N-Ethyl-N-(2-hydroxyethyl)-15-(2-tetrahydropyranyloxy)-11-oxoprosta-5,9,13-trienamide:

To a solution of 250 mg of (5Z,9Z,13E)-(15α)-15-(2-tetrahydropyranyloxy)-11-oxoprosta-5,9,13-trienoic acid (prepared in Reference Example 3) in 10 ml of methylene chloride, 125 μl of triethylamine was added at room temperature under an argon atmosphere. After 0.1 ml of isobutyl chloroformate was then added to the mixture, the resulting mixture was stirred for 15 minutes at the same temperature. To the solution, 88 μl of N-ethylethanolamine was added. After stirring for 15 minutes, the mixture was diluted with methylene chloride, washed with an aqueous sodium bicarbonate solution and a saturated sodium chloride aqueous solution, dried over anhydrous magnesium sulfate and then concentrated under reduced pressure. The residue was purified by column chromatography on silica gel using a

solvent mixture of ethyl acetate and n-hexane as an eluent to give 249 mg of the title compound having the following physical characteristics. Yield, 85 %.

TLC (chloroform : tetrahydrofuran : acetic acid = 10 : 2 : 1): Rf = 0.36;

IR: $\nu$ =3420, 1705, 1620, 1440, 1015, 975 $cm^{-1}$;

NMR: $\delta$=7.53~7.66 (1H, m), 6.09-6.22 (1H, m), 5.3~5.7 (4H, m), 4.6~4.76 (1H, m), 3.96~4.15 (1H, m), 3.58~3.96 (4H, m), 3.28~3.58 (5H, m), 2.75~2.92 (1H, m), 2.6~2.7 (1H, m), 1.19 (3H, t), 0.8~1.0 (3H, m);

Mass: m/e= 489 ($M^+$), 471, 459, 405, 388, 387, 305.

Reference Example 12

(5Z,9Z,13E)-(15α,17α)-N-Ethyl-N-(2-hydroxyethyl)-15-(2-tetrahydropyranyloxy)-11-oxo-17,20-dimethylprosta-5,9,13-trienamide:

The title compound having the following physical characteristics was obtained in a manner similar to Reference Example 9.

Starting material: (5Z,9Z,13E)-(15α,17α)-15-(2-tetrahydropyranyloxy)-11-oxo-17,20-dimethylprosta-5,9,13-trienoic acid (prepared in Reference Example 4(a)), 223 mg.

Yield: 220 mg

TLC (chloroform : tetrahydrofuran : acetic acid = 10 : 2 : 1): Rf = 0.48;

IR: $\nu$ = 3430、1705、1622 $cm^{-1}$;

NMR: $\delta$ = 7.56 (1H, m)、6.14 (1H, m)、5.7 - 5.3 (4H, m)、4.68 (1H, m)、1.0~0.8 (6H, m);

Mass: m/e = 517 ($M^+$)、499。

Reference Example 13

(5Z,9Z,13E)-(15α)-N-Ethyl-N-(2-hydroxyethyl)-15-(2-tetrahydropyranyloxy)-11-oxo-20-methyl-16,18-ethanoprosta-5,9,13-trienamide:

The title compound having the following physical characteristics was obtained in a manner similar to Reference Example 9.

Starting material: (5Z,9Z,13E)-(15α)-15-(2-tetrahydropyranyloxy)-11-oxo-20-methyl-16,18-ethanoprosta-5,9,13-trienoic acid (prepared in Reference Example 4(b)), 202 mg.

Yield: 191 mg

TLC (chloroform : tetrahydrofuran : acetic acid = 10 : 2 : 1): Rf = 0.50;

IR: ν =3430, 1706, 1620;

NMR: δ=7.57 (1H, m), 6.13 (1H, m), 5.7~5.3 (4H, m), 4.67 (1H, m), 1.0~0.8 (3H, m);

Mass: m/e=529 ($M^+$), 511.

Reference Example 14

(5Z,9Z,13E)-(15α)-N-Ethyl-N-(2-hydroxyethyl)-16-(3-chlorophenoxy)-11-oxo-15-(2-tetrahydropyranyloxy)-17,18,19,20-tetranorprosta-5,9,13-trienamide:

The title compound having the following characteristics was obtained in a manner similar to Reference Example 9.

Starting material: (5Z,9Z,13E)-(15α)-16-(3-chlorophenoxy)-11-oxo-15-(2-tetrahydropyranyloxy)-17,18,19,20-tetranorprosta-5,9,13-trienoic acid (prepared in Reference Example 4(c)), 189 mg.

Yield: 162 mg

TLC (chloroform : tetrahydrofuran : acetic acid = 10 : 2 : 1): Rf = 0.46;

IR: $\nu$ =3420, 1705, 1620, 1600 $cm^{-1}$;

NMR: $\delta$=7.62 (1H, dd), 7.30~6.70 (4H, m), 6.20 (1H, dd), 5.78~5.30 (4H, m), 4.68 (1H, m);

Mass: m/e=559 ($M^+$), 543, 541;

## Reference Example 15

(9Z,13E)-(15α)-N-Ethyl-N-(2-hydroxyethyl)-15-(2-tetrahydropyranyloxy)-11-oxoprosta-9,13-dienamide:

The title compound having the following physical characteristics was obtained in a manner similar to Reference Example 9.

Starting material: (9Z,13E)-(15α)-15-(2-tetrahydropyranyloxy)-11-oxoprosta-9,13-dienoic acid (prepared in Reference Example 4(d)), 163 mg.

Yield: 160 mg

TLC (chloroform : tetrahydrofuran : acetic acid = 10 : 2 : 1): Rf = 0.48;

IR: $\nu$ =3420, 1705, 1602 $cm^{-1}$;

NMR: $\delta$=7.56 (1H, m), 6.13 (1H, m), 5.7~5.3 (2H, m), 4.68 (1H, m);

Mass: m/e=491 ($M^+$), 473.

## Reference Example 16

(5Z,13E)-N-Ethyl-N-(2-hydroxyethyl)-9,15-bis(2-tetrahydropyranyloxy)-11-oxoprosta-5,13-dienamide:

The title compound having the following physical characteristics was obtained in a manner similar to Reference Example 9.

Starting material: (5Z,13E)-(9α,15α)-9,15-bis(2-tetrahydropyranyloxy)-11-oxoprosta-5,13-dienoic acid (prepared in Reference Example 7), 103 mg.

Yield: 90 mg

TLC (chloroform : tetrahydrofuran : acetic acid = 10 : 2 : 1): Rf = 0.43;

IR: ν =3430, 2930, 1740, 1645 $cm^{-1}$;

NMR: δ=5.8~5.3 (4H, m), 4.8~4.6 (2H, m), 1.0~0.8 (3H, m).

Reference Example 17

ent-(5Z,13E)-(9β,11β,15αβ)-9,11-dihydroxy-15,20-bis(2-tetrahydropyranyloxy)prosta-5,13-dienoic acid methyl ester

To a solution of 4.49 g of ent-(5Z,13E)-(9β,11β,15αβ)-9,11,15,20-tetrahydroxyprosta-5,13-dienoic acid methyl ester (prepared by the procedure described in Scheme B above) dissolved in 200 ml of methylene chloride, 1.57 g of phenylboric acid was added. The mixture was refluxed and methylene chloride removed over a period of 1.5 hrs. The solution was allowed to cool to room temperature, and 16 ml of 2,3-dihydropyran and pyridinium chloride were added to the solution. The mixture was concentrated under reduced pressure. To a solution of the residue in 25 ml of methanol, 4.9 g of sodium bicarbonate was added. Under ice-cooling, 3.4 ml of a 35 % aqueous solution of hydrogen peroxide was dropped to the solution. The mixture was stirred for 1 hr at room temperature. The mixture obtained was diluted with ethyl acetate and the diluted solution was washed with water, dried over anhydrous magnesium sulphate and then concentrated under reduced pressure to give 3.69 g of the title compound having the following physical characteristics:

TLC(cyclohexane:ethyl acetate = 1:2) : Rf = 0.2;

Mass: m/e = 468, 450, 378, 366, 348, 294.

Reference Example 18

ent-(5Z,13E)-(9β,11β,15αβ)-9-hydroxy-11-benzoyloxy-15,20-bis(2-tetrahydropyranyloxy)prosta-5,13-dienoic acid methyl ester

To a solution of 3.96 g of ent-(5Z,13E)-(9β,11β,15αβ)-9,11-dihydroxy-15,20-bis(2-tetrahydropyranyloxy)-prosta-5,13-dienoic acid methyl ester (prepared in Reference Example 17) dissolved in 150 ml of methylene chloride, 5.4 ml of pyridine was added. To the solution cooled to -40°C, a solution of 1.94 ml of benzoyl chloride in 30 ml of methylene chloride was added dropwise over a period of 45 mins, and the mixture was stirred for 1.5 hrs at the same temperature. After addition of 2 ml of methanol, the reaction mixture was allowed to warm to room temperature. The solution was diluted with ethyl acetate, washed with water, dried over anhydrous magnesium sulphate and then concentrated under reduced pressure. The residue obtained was purified by column chromatography on silica gel using a mixture of cyclohexane and ethyl acetate (2:1) as eluent to give 4.03 g of the title compound having the following physical characteristics:

TLC(cyclohexane:ethyl acetate = 2:1) : Rf = 0.45;

Mass: m/e = 470, 400.

Reference Example 19

ent-(5Z,13E)-(9β,11β,15αβ)-9,15,20-tris(2-tetrahydropyranyloxy)-11-hydroxyprosta-5,13-dienoic acid

To a solution of 4.03 g of ent-(5Z,13E)-(9β,11β,15αβ)-9-hydroxy-11-benzoyloxy-15,20-bis(2-tetrahydropyranyloxy)-prosta-5,13-dienoic acid methyl ester (prepared in Reference Example 18) dissolved in 30 ml of methylene chloride, 0.67 ml of 2,3-dihydropyran and a catalytic amount of p-toluenesulphonic acid were added, and the mixture was stirred for 40 min. The solution was worked up by the addition of drops of triethylamine and concentrated under reduced pressure. The residue obtained was dissolved in 70 ml of methanol. To the solution, 30 ml of a 1N aqueous solution of sodium hydroxide was added, and the mixture was stirred for 3 hrs at 50°C. Under ice-cooling, the mixture was acidified with hydrochloric acid, and then extracted with ethyl acetate. The extract was washed with water, dried over anhydrous magnesium sulphate and concentrated under reduced pressure. The residue was purified by column chromatography on silica gel using a mixture of cyclohexane and ethyl acetate (1:2) as eluent to give 2.92 g of the title compound having the following physical characteristics:

TLC(cyclohexane:ethyl acetate = 1:2) : Rf = 0.15;

Mass: m/e = 436, 418.

Reference Example 20

ent-(5Z,13E)-(9β,15αβ)-9,15,20-tris(2-tetrahydropyranyloxy)-11-oxoprosta-5,13-dienoic acid

To a -20°C cooled solution of 2.9 g of ent-(5Z,13E)-(9β,11β,15αβ)-9,15,20-tris(2-tetrahydropyranyloxy)-11-hydroxyprosta-5,13-dienoic acid (prepared in Reference Example 19) in 50 ml of acetone, 8.76 ml of Jones reagent (2.76N) was added dropwise, and the mixture was worked up by adding 2 ml of isopropanol. The mixture was diluted with ethyl acetate, and the diluted solution was washed with water, dried over anhydrous magnesium sulphate and concentrated under reduced pressure. The residue was purified by column chromatography on silica gel using a mixture of cyclohexane and ethyl acetate (1:1) as eluent to give 2.2 g of the title compound having the following physical characteristics:

TLC(cyclohexane:ethyl acetate = 1:2) : Rf = 0.4;

NMR: δ = 5.75-5.30(4H,m), 4.70-4.50(3H,m);

Mass: m/e = 434, 416, 350, 332.

Reference Example 21

ent-(5Z,9Z,12EZ,14EZ)-11-oxo-20-hydroxyprosta-5,9,12,14-tetraenoic acid

To a solution of 2.1g of ent-(5Z,13E)-(9β,15α/β)-9,15,20-tris(2-tetrahydropyranyloxy)-11-oxoprosta-5,13-dienoic acid (prepared in Reference Example 20) dissolved in 100 ml of tetrahydrofuran, 20.3 ml of 1N hydrochloric acid was added. The solution was stirred for 1 hr at 70°C. The solution was diluted with ethyl acetate, and the diluted solution was washed with water, dried over anhydrous magnesium sulphate and concentrated under reduced pressure. The residue was purified by column chromatography on silica gel using a mixture of cyclohexane and ethyl acetate (1:2) as eluent to give 422 mg of the title compound having the following physical characteristics:

TLC(cyclohexane:ethyl acetate=1:2): Rf=0.2;

NMR: $\delta$=7.48(1H,dd), 6.94(1H,d), 6.40-6.12 (3H,m), 5.54-5.30(2H,m), 3.67(2H,t), 2.70-2.56(1H,m);

Mass: m/e=332($M^+$), 314, 245.

Example 1

(5Z,9Z,13E)-(15α)-N-(2-Hydroxyethyl)-15-hydroxy-11-oxoprosta-5,9,13-trienamide (i.e., 9-Deoxy-Δ9-$PGD_2$ ethanolamide):

183 mg of the $PGD_2$ amide prepared in Reference Example 9 was dissolved in 0.5 ml of tetrahydrofuran and 5 ml of 65 % acetic acid was added to the resulting solution. The mixture was stirred at 80°C for 7 minutes. After allowing the mixture to cool, ethyl acetate was added. The diluted solution obtained was washed with an aqueous sodium bicarbonate solution and a saturated sodium chloride aqueous solution,

dried over anhydrous magnesium sulfate and concentrated under reduced pressure. The residue was purified by column chromatography on silica gel using a solvent mixture of ethyl acetate and methanol as an eluent to give 108 mg of the title compound having the following physical characteristics. Yield, 72%.

TLC (chloroform : tetrahydrofuran : acetic acid = 10 : 2 : 1): Rf = 0.16;

IR: $\nu$ :3320、1700、1640、1240、1050、975$cm^{-1}$;

NMR: $\delta$=7.60(1H, dd)、6.18(1H, dd)、5.3~5.8(4H, m)、4.0~4.2(1H, m)、3.70(2H, t)、3.41(2H, t)、2.8~2.95(1H, m)、2.6~2.75(1H, m)、0.8~1.0(3H, m)。

Mass: m/e=377($M^+$)、359、288、277、170。

## Example 2

(5Z,9Z,13E)-(15$\alpha$)-N-(1,3-Dihydroxy-2-methylpropan-2-yl)-15-hydroxy-11-oxoprosta-5,9,13-trienamide (i.e., 9-Deoxy-$\Delta^9$-$PGD_2$ 2-methyl-1,3-propanediol-2-amide):

In 0.5 ml of tetrahydrofuran, 182 mg of the $PGD_2$ amide prepared in Reference Example 10 was dissolved and 5 ml of 65 % acetic acid was added to the solution. The mixture was stirred at 80°C for 8 minutes. After allowing to cool, ethyl acetate

was added to the solution. The diluted solution was washed with an aqueous sodium bicarbonate solution and a saturated sodium chloride aqueous solution, dried over anhydrous magnesium sulfate and concentrated under reduced pressure. The residue was purified by column chromatography on silica gel using a solvent mixture of ethyl acetate and methanol as an eluent to give 74 mg of the title compound having the following physical characteristics. Yield, 49%.

TLC (chloroform : tetrahydrofuran : acetic acid = 10 : 2 : 1): Rf = 0.10;

IR: $\nu$ = 3350, 1700, 1640, 1540, 1450, 1245, 1040, 975 $cm^{-1}$;

NMR: $\delta$ = 7.60 (1H, dd), 6.16 (1H, dd), 5.3~5.68 (4H, m), 4.0~4.2 (1H, m), 3.52~3.76 (4H, m), 2.8~2.92 (1H, m), 2.61~2.70 (1H, m), 1.22 (3H, s), 0.8~1.0 (3H, m);

Mass: m/e = 421 ($M^+$), 403, 390, 372, 354, 321.

## Example 3

(5Z,9Z,13E)-(15α)-N-Ethyl-N-(2-hydroxyethyl)-15-hydroxy-11-oxoprosta-5,9,13-trienamide (i.e., 9-Deoxy-$\Delta^9PGD_2$ N-ethylethanolamide):

In 0.5 ml of tetrahydrofuran, 230 mg of the $PGD_2$ amide prepared

in Reference Example 11 was dissolved and 5 ml of 65% acetic acid was added to the solution. The mixture was stirred at 80°C for 8 minutes. After allowing to cool, ethyl acetate was added to the solution. The diluted solution was washed with an aqueous sodium bicarbonate solution and a saturated sodium chloride aqueous solution, dried over anhydrous magnesium sulfate and concentrated under reduced pressure. The residue was purified by column chromatography on silica gel using a solvent mixture of ethyl acetate and methanol as an eluent to give 133 mg of the title compound having the following physical characteristics. Yield, 70%.

TLC (chloroform : tetrahydrofuran : acetic acid = 10 : 2 : 1): Rf = 0.33;

IR: ν =3410、1705、1620、1455、1240、1050、965 $cm^{-1}$;

NMR: δ=7.59 (1H, dd)、6.14 (1H, dd)、5.28～5.80 (4H, m)、4.0～4.2 (1H, m)、3.64～3.85 (2H, m)、3.27～3.56 (4H, m)、2.8～2.95 (1H, m)、2.6～2.73 (1H, m)、1.18 (3H, t)、0.8～1.0 (3H, m);

Mass: m/e=405 ($M^+$)、387、334、316、305.

## Example 4

(5Z,9Z,13E)-(15α,17α)-N-Ethyl-N-(2-hydroxyethyl)-11-oxo-17,20-dimethyl-15-hydroxyprosta-5,9,13-trienamide (i.e., 9-Deoxy-$\Delta^9$-17,20-dimethyl-$PGD_2$ N-ethylethanolamide):

The title compound having the following physical characteristics was obtained in a manner similar to Example 3.

Starting material: $PGD_2$ amide prepared in Reference Example 12, 217 mg.

Yield: 120 mg

TLC (chloroform : tetrahydrofuran : acetic acid = 10 : 2 : 1): Rf = 0.34;

IR: $\nu$ =3410、1706、1622;

NMR: $\delta$=7.60 (1H, dd)、6.15 (1H, dd)、5.80~5.26 (4H, m)、1.0~0.8 (6H, m);

Mass: m/e=433 ($M^+$)、415。

## Example 5

(5Z,9Z,13E)-(15α)-N-Ethyl-N-(2-hydroxyethyl)-15-hydroxy-11-oxo-20-methyl-16,18-ethanoprosta-5,9,13-trienamide (i.e., 9-Deoxy-$\Delta^9$-16,18-ethano-20-methyl-$PGD_2$ N-ethylethanolamide):

The title compound having the following physical characteristics was obtained in a manner similar to Example 3.

Starting material: $PGD_2$ amide prepared in Reference Example 13, 184 mg.

Yield: 110 mg

TLC (chloroform : tetrahydrofuran : acetic acid = 10 : 2 : 1): Rf = 0.35;

IR: ν =3410, 1706, 1620 $cm^{-1}$;

NMR: δ=7.59 (1H, dd), 6.14 (1H, dd), 5.82-5.28 (4H, m), 1.0~0.8 (3H, m);

Mass: m/e=445 ($M^+$), 427.

## Example 6

(5Z,9Z,13E)-(15α)-N-Ethyl-N-(2-hydroxyethyl)-16-(3-chlorophenoxy)-15-hydroxy-11-oxo-17,18,19,20-tetranorprosta-5,9,-13-trienamide (i.e., 9-Deoxy-$\Delta^9$-16-(3-chlorophenoxy)-17,18,19,20-tetranor-$PGD_2$ N-ethylethanolamide):

The title compound having the following physical characteristics was obtained in a manner similar to Example 3.

Starting material: $PGD_2$ amide prepared in Reference Example 14, 151 mg.

Yield: 86 mg

TLC (chloroform : tetrahydrofuran : acetic acid = 10 : 2 : 1): Rf = 0.30;

IR: $\nu$ =3410, 1705, 1620, 1600 $cm^{-1}$;
NMR: $\delta$=7.62 (1H, dd), 7.30~6.70 (4H, m), 6.20 (1H, dd), 5.80~5.30 (4H, m);
Mass: m/e=475 ($M^+$), 459, 457.

Example 7

(9Z,13E)-(15α)-N-Ethyl-N-(2-hydroxyethyl)-15-hydroxy-11-oxoprosta-9,13-dienamide (i.e., 9-Deoxy-$\Delta^9$-$PGD_1$ N-ethylethanolamide):

The title compound having the following physical characteristics was obtained in a manner similar to Example 3.

Starting material: $PGD_1$ amide prepared in Reference Example 15, 155 mg.

Yield: 95 mg

TLC (chloroform : tetrahydrofuran : acetic acid = 10 : 2 : 1): Rf = 0.35;

IR: $\nu$ =3410, 1706, 1602 $cm^{-1}$;
NMR: $\delta$=7.58 (1H, dd), 6.13 (1H, dd), 5.70~5.30 (2H, m);
Mass: m/e=407 ($M^+$), 389.

Example 8

(5Z,13E)-N-Ethyl-N-(2-hydroxyethyl)-9,15-dihydroxy-11-oxoprosta-5,13-dienamide (i.e., $PGD_2$ N-ethylethanolamide):

The title compound having the following physical characteristics was obtained in a manner similar to Example

3.

Starting material: $PGD_2$ amide prepared in Reference Example 16, 82 mg.

Yield: 63 mg

IR: ν =3400, 2925, 1735, 1620 $cm^{-1}$;

NMR: δ=5.75-5.30 (4H, m), 4.52~4.42 (1H, m), 1.0~0.8 (3H, m).

## Example 9

(5Z,9Z,12EZ,14EZ)-N-Ethyl-N-(2-hydroxyethyl)-11-oxoprosta-5,9,12,14-tetraenamide (i.e., 9,15-Dideoxy-13,14-dihydro-$\Delta^{9,12,14}$-$PGD_2$ N-ethylethanolamide):

To a solution of 316 mg of (5Z,9Z,12EZ,14EZ)-11-oxo-prosta-5,9,12,14-tetraenoic acid (prepared in Reference Example 8) in 15 ml of methylene chloride, 0.2 ml of triethylamine was added and 0.16 ml of isobutyl chloroformate was further added thereto. After stirring for 15 minutes, 0.16 ml of N-ethylethanolamine was added to the mixture followed by further stirring for 15 minutes. To the solution, 150 ml of methylene chloride was added. The diluted solution was washed with an aqueous sodium bicarbonate solution and a saturated sodium chloride aqueous solution, dried over anhydrous magnesium sulfate and concentrated under reduced pressure. The residue was purified by column chromatography on silica gel using a solvent mixture of ethyl

acetate and n-hexane (1 : 2) as an eluent to give 290 mg of the title compound having the following physical characteristics:

TLC (chloroform : tetrahydrofuran : acetic acid = 10 : 2 : 1): Rf = 0.52;

IR: $\nu$ =3430、2930、1695、1630 $cm^{-1}$;

NMR: $\delta$=7.55-7.44 (1H, m)、6.95 (1H, d)、 6.5~6.2 (3H, m)、1.0~0.8 (3H, m);

Mass: m/e=387 ($M^+$)。

Example 9(a)

(5Z,9Z,12EZ,14EZ)-N-(1,3-dihydroxy-2-methylpropan-2-yl)-11-oxoprosta-5,9,12,14-tetraenamide

[i.e. 9,15-dideoxy-13,14-dihydro-$\Delta^{9,12,14}$-$PGD_2$ 2-methyl-1,3-propanediol-2-amide]

By the same procedure as Example 9, the title compound having the following physical characteristics, was obtained:

Starting materials: 112 mg of acid prepared in Reference Example 8; 2-amino-2-methylpropane-1,3-diol (57mg).

Yield: 115 mg;

TLC (Ethyl acetate): Rf = 0.24;

IR: $\nu$ = 3320, 2940, 1736, 1690-1620, 1056 $cm^{-1}$;

NMR: $\delta$= 7.47(1H,dd), 6.94(1H,d), 6.42-6.21(3H,m), 5.94(1H,s), 5.53-5.24(2H,m), 5.77-3.54(5H,q), 1.22(3H,s), 0.90(3H,t);

Mass: m/e = 403($M^+$), 385, 372, 354.

Example 9(b)

ent-(5Z,9Z,12EZ,14EZ)-N-tris(hydroxymethyl)methyl-11-oxo-20-hydroxyprosta-5,9,12,14-tetraenamide [i.e. ent-9,15-dideoxy-13,14-dihydro-$\Delta^{5,9,12,14}$-$PGD_2$ tris(hydroxymethyl)methylamide]

By the same procedure as Example 9, the title compound having the following physical characteristics was obtained:

Starting material: 256 mg of acid prepared in Reference Example 21 and 139 mg of 1,3-dihydroxy-2-hydroxymethyl-2-aminopropane

Yields: 315 mg;

TLC(ethyl acetate:methanol=20:1): Rf=0.1;

IR: $\nu$=3600-3100, 1690, 1630,1550, 1440, 1210, 1050, 1030 $cm^{-1}$;

NMR: $\delta$=7.56-7.46(1H, m), 6.95(1H, d), 6.56 (2H, s), 6.46-6.16(2H, m), 5.58-5.38 (2H, m), 3.70-3.56(9H, m);

Mass: m/e=417, 386, 368, 330, 304, 286.

Example 9(c)

(5Z,13E)-(9α,15α)-N-(1,3-dihydroxy-2-methylpropan-2-yl)-9,15-dihydroxy-11-oxoprosta-5,13-dienamide
[i.e. $PGD_2$ 2-methyl-1,3-propanediol-2-amide]

By the same procedure as Example 9, the title compound having the following physical characteristics was obtained:

Starting material: 356 mg of $PGD_2$ and 170 mg of 2-amino-2-methyl-1,3-propanediol

Yields: 391 mg;

TLC(methylene chloride:methanol = 10:1); Rf=0.41;

IR: $\nu$=3350, 1730, 1690, 1650, 1630 $cm^{-1}$;

NMR: $\delta$ =6.26(1H,s), 5.4-5.7(4H,m), 4.36-4.5(1H,m), 3.95-4.15(1H,m), 3.5-3.8(4H,m), 3.08(2H,q), 2.82(1H,dd), 0.86(3H,t);

Mass: m/e=421, 403, 385.

The present invention includes within its scope pharmaceutical compositions which comprise at least one prostaglandin D analogue of general formula (I) or enantiomer thereof or cyclodextrin clathrate thereof in association with a pharmaceutically acceptable carrier or coating.

In clinical practice the compounds of the present invention will normally be administered systemically or partially, usually by oral or parenteral (e.g. intravenous, subcutaneous or intramuscular) administration.

The dose to be administered is determined depending upon, for example, age, body weight, symptoms, the desired therapeutic effect, the route of administration, and the duration of the treatment.

In the human adult, the doses per person are generally between 5 mg and 500 mg by oral administration, and between 500 μg and 50 mg by parenteral, preferably intravenous administration and can be administered up to several times per day, for the prevention or therapy (including therapy to secure remission) of leukemia or solid cancer, or of infection or leukocyto-penia, or to synchronize the cell cycle of tumour cells.

As mentioned above, the doses to be used depend on various factors. Therefore, there may be cases in which doses greater than the ranges specified above, or lower than the ranges specified above, may be used.

In the present invention, solid compositions for oral administration include compressed tablets, pills, dispersible powders and granules. In such solid compositions, one or more of the active compound(s) is, or are, admixed, with at least one inert diluent such as lactose, mannitol, glucose, hydroxypropyl cellulose, microcrystalline cellulose, starch,

polyvinylpyrrolidone or magnesium metasilicate aluminate. The compositions may also comprise, as is normal practice, additional substances other than inert diluents, e.g. lubricating agents such as magnesium stearate, and disintegrating agents, such as cellulose calcium gluconate. The tablets or pills may, if desired, be made into enteric film-coated or gastric film-coated, tablets or pills such as sugar-coated, gelatin-coated,hydroxypropylcellulose-coated or hydroxypropylmethyl-cellulose phthalate-coated tablets or pills; two or more layers may be used. The compositions for oral administration also include capsules of absorbable material such as gelatin.

Liquid compositions for oral administration include pharmaceutically acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or more active substances.

Preparations according to the invention for parenteral administration include sterile aqueous or non-aqueous solutions, suspensions or emulsions. Examples of aqueous

solvents or suspending media are distilled water for injection and physiological salt solution. Examples of non-aqueous solvents or suspending media are propylene glycol, polyethylene glycol, vegetable oils such as olive oil, alcohols such as ethanol, and Polysorbate 80 (registered Trade Mark). These compositions may also include adjuvants such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions include, for parenteral administration, liquids for external use, and endermic liniments such as ointments; suppositories for rectal administration; and pessaries for vaginal administration.

The following Examples illustrate pharmaceutical compositions according to the invention:

## Example 10

A solution of 1 g of 9-deoxy-$\Delta^9$-$PGD_2$ ethanolamide (prepared in Example 1) in 5 ml of ethanol was well mixed with 5 g of microcrystalline cellulose and dried sufficiently. To the resulting mixture, 100 mg of magnesium stearate, 20 mg of silicon dioxide, 10 mg of talc and 200 mg of cellulose calcium gluconate (CCG) were admixed and then microcrystalline cellulose was added to make the total weight 10 g. The resulting mixture was well mixed to make it homogeneous, and then tabletted in conventional manner to give 100 tablets each containing 10 mg of the active material.

## Example 11

α- and ß-Cyclodextrin clathrate was prepared by the

following procedure: To a solution of 2.4 g of α-cyclodextrin and 1 g of β-cyclodextrin in 300 ml of water, 100 mg of $PGD_2$ ethanolamide (prepared in Example 1) was added, and the mixture was thoroughly stirred. The resulting solution was concentrated under reduced pressure to yield the clathrate of 9-deoxy-$\Delta^9$-$PGD_2$ ethanolamide. The clathrate was dissolved in 150 ml of distilled water for injection. The aqueous solution was filtered to sterilize it, pipetted into 5 ml ampoules to give 1.5 ml per ampoule, and freeze dried to give 100 ampoules for injection each containing 1 mg of the active material.

Example 12

Tablets and solid preparations for injection each containing the compounds prepared in Examples 2 through 9(c) as the active ingredients were obtained by the procedures of Examples 11 and 12.

## CLAIMS

1. A prostaglandin D analogue of the formula:

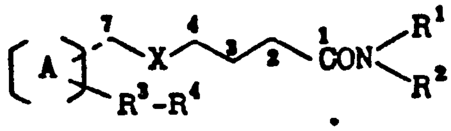

(I)

wherein the symbol [A] represents a group of the formula:

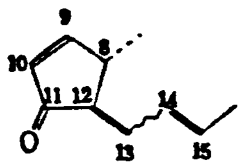

(II)

,

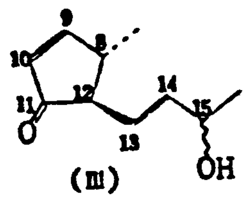

(III)

or

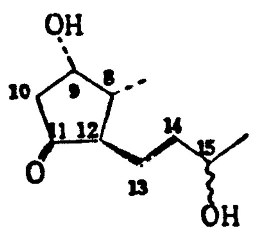

(IV)

wherein the double bonds between $C_{12}$-$C_{13}$ and $C_{14}$-$C_{15}$ in the formula (II), which may be in the same or different configurations are trans, cis or a mixture thereof, the double bond between $C_9$-$C_{10}$ is cis, the double bonds between $C_9$-$C_{10}$ and $C_{13}$-$C_{14}$ in the formula (III) are, respectively, cis and trans and the double bond between $C_{13}$-$C_{14}$ in the formula (IV) is trans, X represents an ethylene group or cis-vinylene group, $R^1$ represents a hydrogen atom or a straight-chain or branched-chain alkyl group of 1 to 4 carbon atoms, $R^2$ represents a straight-chain or branched-chain alkyl group of 2 to 6 carbon atoms substituted by 1 to 3 hydroxy groups, $R^3$ represents a single bond or a straight-chain or branched-chain alkylene group of 1 to 5 carbon atoms, $R^4$ represents a straight-chain or branched-chain alkyl group of 1 to 8 carbon atoms unsubstituted or substituted by at least one hydroxy group, a cycloalkyl

group of 4 to 7 carbon atoms unsubstituted or substituted by at least one straight-chain or branched-chain alkyl group of 1 to 8 carbon atoms, or a phenyl or phenoxy group unsubstituted or substituted by at least one halogen atom, trifluoromethyl group or straight-chain or branched-chain alkyl group of 1 to 4 carbon atoms, with the proviso that when $R^3$ represents a single bond, $R^4$ does not represent an unsubstituted or substituted phenoxy group], or an enantiomer thereof or a cyclodextrin clathrate thereof.

2. A prostaglandin D analogue according to claim 1 wherein $R^1$ represents a hydrogen atom, or a methyl or ethyl group.

3. A prostaglandin D analogue according to claim 1 or 2 wherein $R^2$ represents 2-hydroxyethyl, 1,3-dihydroxymethyl-2-methylpropan-2-yl, tris(hydroxymethyl)methyl or 1-hydroxymethylethyl.

4. A prostaglandin D analogue according to claim 1, 2 or 3 wherein $R^3$ represents a single bond or a methylene or ethylene group.

5. A prostaglandin D analogue according to any one of claims 1 to 4 wherein $R^4$ represents a butyl, pentyl or hexyl group unsubstituted or substituted by one or two methyl or ethyl groups and/or one or two hydroxy groups; a cyclobutyl, cyclopentyl or cyclohexyl group unsubstituted or substituted by one methyl, ethyl, propyl or butyl group; or a phenyl or phenoxy group unsubstituted or substituted by at least one chlorine atom, trifluoromethyl, methyl or ethyl group.

6. A prostaglandin analogue according to any one of claims 1 to 5 wherein, in general formulae (III) and (IV), the hydroxy group attached to the carbon atom at the 15-position is in α-configuration.

7. A compound according to claim 1 which is (5Z,9Z,12EZ,14EZ)-N-ethyl-N-(2-hydroxyethyl)-11-oxoprosta-5,9,12,14-tetraenamide, (5Z,9Z,13E)-(15α)-N-(2-hydroxyethyl)-15-hydroxy-11-oxoprosta-5,9,13-trienamide, (5Z,9Z,13E)-(15α)-N-(1,3-dihydroxy-2-methylpropan-2-yl)-15-hydroxy-11-oxoprosta-5,9,13-trienamide, (5Z,9Z,13E)-(15α)-N-ethyl-N-(2-hydroxyethyl)-15-hydroxy-11-oxoprosta-5,9,13-trienamide, (9Z,13E)-N-ethyl-N-(2-hydroxyethyl)-15-hydroxy-11-oxoprosta-9,13-dienamide, (5Z,9Z,13E)-(15α,17α)-N-ethyl-N-(2-hydroxyethyl)-15-hydroxy-17,20-dimethylprota-5,9,13-trienamide, (5Z,9Z,13E)-(15α)-N-ethyl-N-(2-hydroxyethyl)-15-hydroxy-11-oxo-20-methyl-16,18-ethanoprosta-5,9,13-trienamide, (5Z,9Z,13E)-(15α)-N-ethyl-N-(2-hydroxyethyl)-16-(3-chlorophenoxy)-15-hydroxy-11-oxo-17,18,19,20-tetranorprosta-5,9,13-trienamide, (5Z,13E)-N-ethyl-N-(2-hydroxyethyl)-9,15-dihydroxy-11-oxoprosta-5,13-dienamide, (5Z,9Z,12EZ,14EZ)-N-(1,3-dihydroxy-2-methyl-propan-2-yl)-11-oxoprosta-5,9,12,14-tetraenamide, ent-(5Z,9Z,12EZ,14EZ)-N-tris(hydroxymethyl)methyl-11-oxo-20-hydroxyprosta-5,9,12,14-tetraenamide or (5Z,13E)-(9α,15α)-N-(1,3-dihydroxy-2-methyl-propan-2-yl)-9,15-dihydroxy-11-oxoprosta-5,13-dienamide or a cyclodextrin clathrate thereof.

8. A process for the preparation of prostaglandin D analogues of general formula I depicted in claim 1, or an enantiomer thereof, wherein the various symbols are as defined in claim 1, which comprises

(A) when the prostaglandin D analogues are of the general formula:

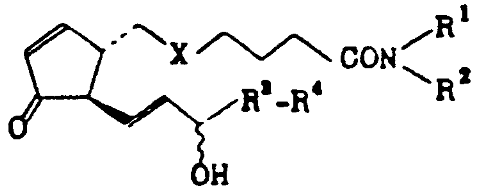

(IA)

(wherein all the symbols are as defined in claim 1) the hydrolysis under acidic conditions of a compound of the general formula:

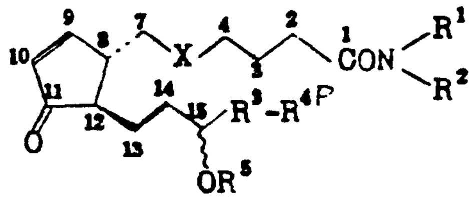

(V)

(wherein $R^5$ represents a 2-tetrahydropyranyl group or 2-tetrahydrofuranyl group either unsubstituted or substituted by at least alkyl group or a 1-ethoxyethyl group, $R^{4p}$ represents a group $R^4$ as defined in claim 1 in which, when $R^4$ is a straight-chain or branched-chain alkyl group of 1 to 8 carbon atoms substituted by at least one hydroxy group, the or each hydroxy group is protected by a group $R^5$ as hereinbefore defined and the other symbols are as defined in claim 1) to convert hydroxy groups protected by a group $R^5$ to hydroxy groups;

(B) when the prostaglandin D analogues are of the general formula:

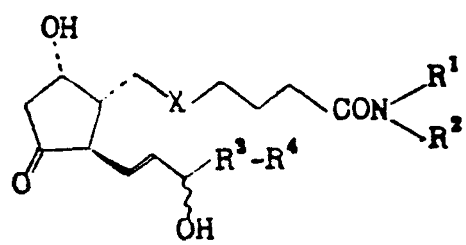

(Ie)

(wherein all the symbols are as defined in claim 1) the hydrolysis under acidic conditions of a compound of the general formula:

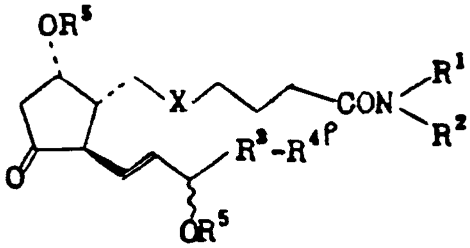

(VII)

(wherein $R^5$ and $R^{4p}$ are as hereinbefore defined and the other symbols are as defined in claim 1) to convert hydroxy groups protected by a group $R^5$ to hydroxy groups;

(C) when the prostaglandin D analogues are of the general formula:

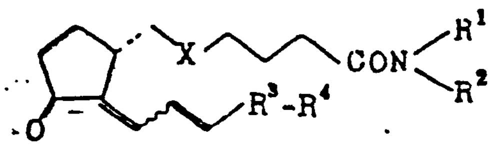

(Ia)

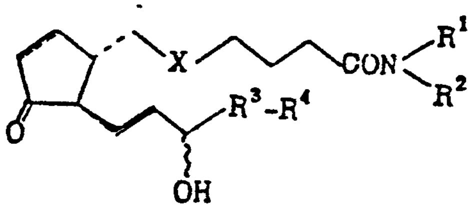

(Ib)

or

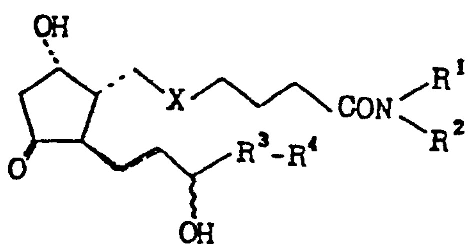

(Ic)

(wherein all the symbols are as defined in claim 1) the reaction of a compound of the general formula:

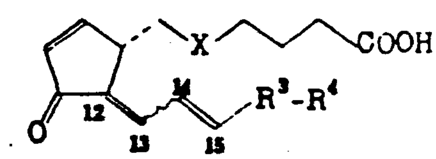

(X)

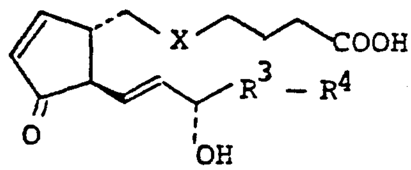

(XXXVII)

or

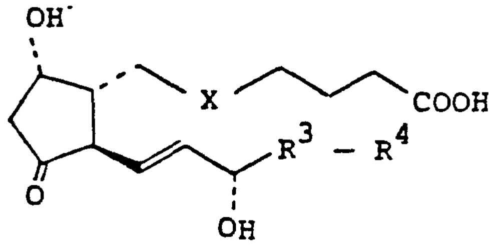

(XXXV)

(wherein all the symbols are as defined in claim 1) with a compound of the general formula:

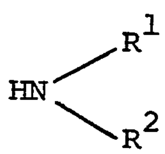

(IX)

(wherein $R^1$ and $R^2$ are as defined in claim 1); or

(D) when the prostaglandin D analogues are of the general formula:

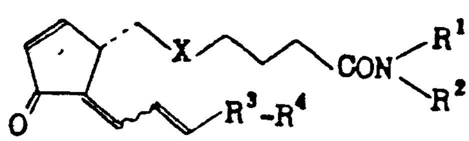

(Iα)

(wherein all the symbols are as defined in claim 1) the reaction of a compound of the general formula:

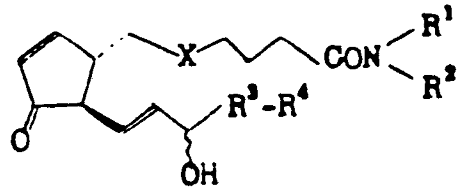

(Ib)

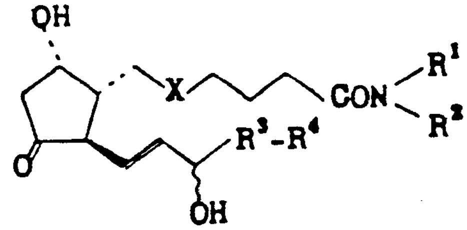

(Ic)

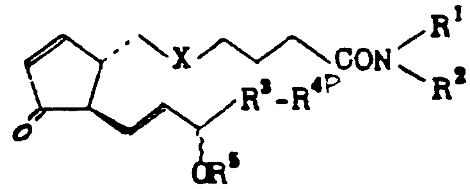

(V)

or

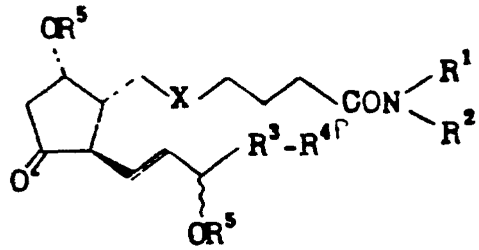

(VI)

(wherein $R^5$ and $R^{4p}$ are as hereinbefore defined and the other symbols are as defined in claim 1) with an aqueous acid at the reflux temperature; optionally followed by the step of converting a prostaglandin D analogue obtained into a cyclodextrin clathrate thereof.

9. A prostaglandin D analogue according to claim 1 or an enantiomer thereof or cyclodextrin clathrate thereof, when prepared by a process claimed in claim 8.

10. A pharmaceutical composition which comprises, as active ingredient, a prostaglandin D analogue of the general formula (I) depicted in claim 1, or an enantiomer thereof or cyclodextrin clathrate thereof, in association with a pharmaceutical carrier or coating.

11. A prostaglandin D analogue of the general formula (I) depicted in claim 1 or an enantiomer thereof or cyclodextrin clathrate thereof for use in therapy.

12. A compound of formula (V) or (VI) wherein the various symbols are as defined in claims 1 and 8.

CLAIMS FOR AUSTRIA

1. A process for the preparation of a prostaglandin D analogue of the formula:

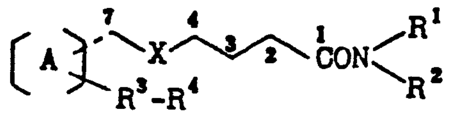

(I)

wherein the symbol [A] represents a group of the formula:

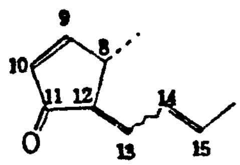

(II)

,

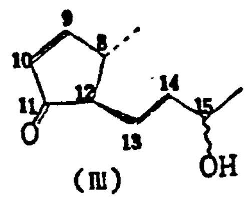

(III)

or

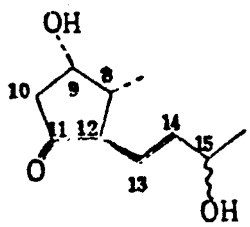

(IV)

wherein the double bonds between $C_{12}$-$C_{13}$ and $C_{14}$-$C_{15}$ in the formula (II), which may be in the same or different configurations are trans, cis or a mixture thereof, the double bond between $C_9$-$C_{10}$ is cis, the double bonds between $C_9$-$C_{10}$ and $C_{13}$-$C_{14}$ in the formula (III) are, respectively, cis and trans and the double bond between $C_{13}$-$C_{14}$ in the formula (IV) is trans, X represents an ethylene group or cis-vinylene group, $R^1$ represents a hydrogen atom or a straight-chain or branched-chain alkyl group of 1 to 4 carbon atoms, $R^2$ represents a straight-chain or branched-chain alkyl group of 2 to 6 carbon atoms substituted by 1 to 3 hydroxy groups, $R^3$ represents a single bond or a straight-chain or branched-chain alkylene group of 1 to 5 carbon atoms, $R^4$ represents a straight-chain or branched-chain alkyl group of 1 to 8 carbon atoms unsubstituted or substituted by at least one hydroxy group, a cycloalkyl

group of 4 to 7 carbon atoms unsubstituted or substituted by at least one straight-chain or branched-chain alkyl group of 1 to 8 carbon atoms, or a phenyl or phenoxy group unsubstituted or substituted by at least one halogen atom, trifluoromethyl group or straight-chain or branched-chain alkyl group of 1 to 4 carbon atoms, with the proviso that when $R^3$ represents a single bond, $R^4$ does not represent an unsubstituted or substituted phenoxy group], or an enantiomer thereof or a cyclodextrin clathrate thereof, characterised in that it is prepared by a process which comprises:
(A) when the prostaglandin D analogues are of the general formula:

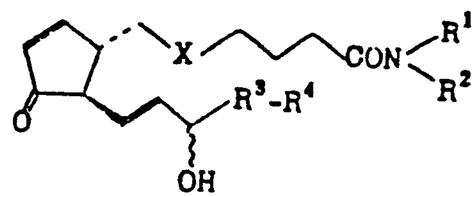

(Ib)

(wherein all the symbols are as hereinbefore defined) the hydrolysis under acidic conditions of a compound of the general formula:

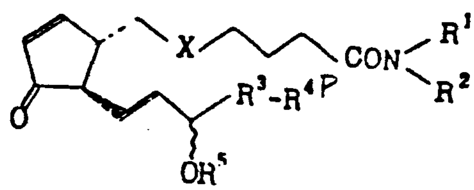

(V)

(wherein $R^5$ represents a 2-tetrahydropyranyl group or 2-tetrahydrofuranyl group either unsubstituted or substituted by at least alkyl group or a 1-ethoxyethyl group, $R^{4P}$ represents a group $R^4$ as hereinbefore defined in which, when $R^4$ is a straight-chain or branched-chain alkyl group of 1 to 8 carbon atoms substituted by at

least one hydroxy group, the or each hydroxy group is protected by a group $R^5$ as hereinbefore defined and the other symbols are as hereinbefore defined) to convert hydroxy groups protected by a group $R^5$ to hydroxy groups;

(B) when the prostaglandin D analogues are of the general formula:

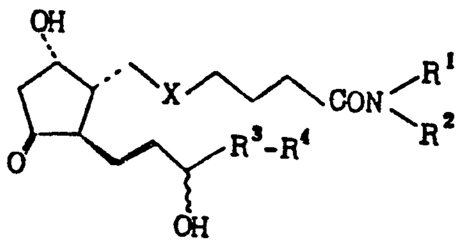

(Ic)

(wherein all the symbols are as hereinbefore defined) the hydrolysis under acidic conditions of a compound of the general formula:

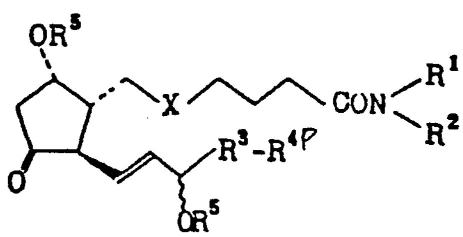

(VI)

(wherein all of the symbols are as hereinbefore defined) to convert hydroxy groups protected by a group $R^5$ to hydroxy groups;

(C) when the prostaglandin D analogues are of the general formula:

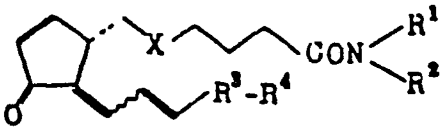

(Ia)

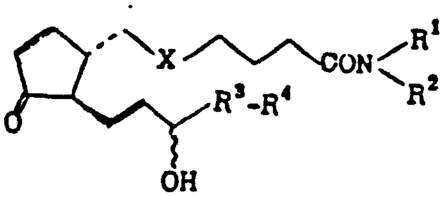

(Ib)

and

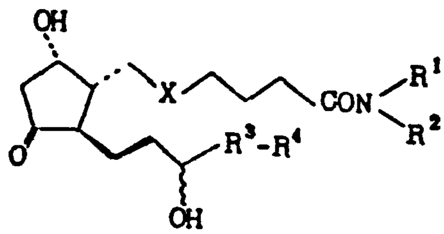

(Ic)

(wherein all the symbols are as hereinbefore defined) the reaction of a compound of the general formula:

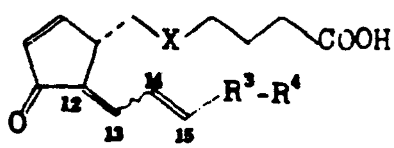

(X)

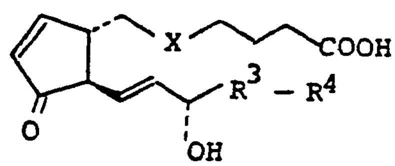

(XXXVII)

or

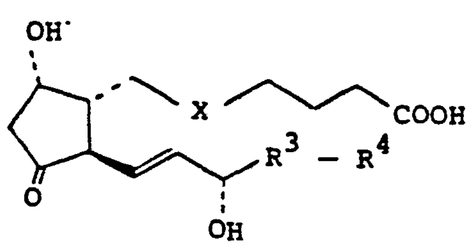

(XXXV)

(wherein all the symbols are as hereinbefore defined) with a compound of the general formula:

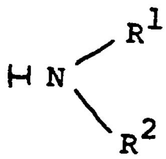

(IX)

(wherein $R^1$ and $R^2$ are as hereinbefore defined); or

(D) when the prostaglandin D analogues are of the general formula:

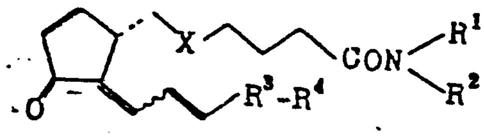

(Ia)

(wherein all the symbols are as hereinbefore defined) the reaction of a compound of the general formula:

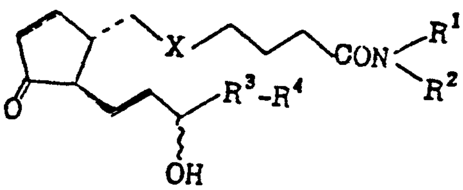

(Ib)

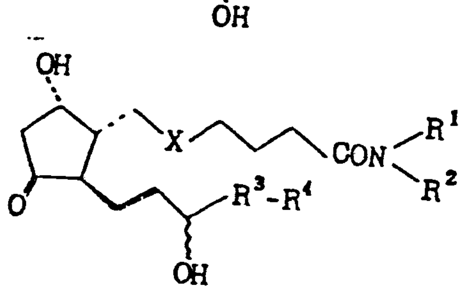

(Ic)

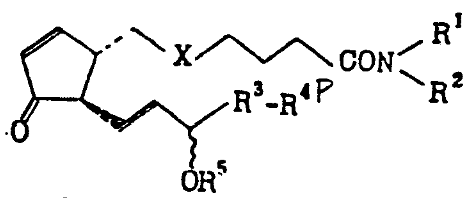

(V)

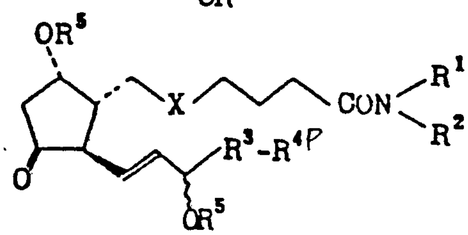

(VI)

(wherein all of the symbols are as hereinbefore defined) with an aqueous acid at the reflux temperature; optionally followed by the step of converting a prostaglandin D analogue obtained into a cyclodextrin clathrate thereof.

2. A process according to claim 1 characterised in that the symbol $R^5$ represents a 2-tetrahydropyranyl group.

3. A process according to claim 1 or 2 characterised in that the hydrolysis under acidic conditions in process (A) or (B) is carried out using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran or a mixture of p-toluenesulphonic acid and methanol.

4 A process according to claim 1 or 2 characterised in that process (D) is carried out in tetrahydrofuran using 1N hydrochloric acid at the reflux temperature of the reaction mixture.

5. A process according to claim 1 characterised in that, in process (C), the compound of general formula (X), (XXXVII) or (XXXV) is reacted with chloroformic acid, isobutyl chloroformate or trimethylacetyl chloride in an inert organic solvent in the presence of a base at a temperature of from $0^{\circ}C$ to $40^{\circ}C$, and then the compound thus obtained is reacted with a compound of general formula (IX).

European Patent Office

**EUROPEAN SEARCH REPORT**

0172963

EP 84 30 5960

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | EP-A-0 098 141 (ONO PHARMACEUTICAL COMPANY)<br>--- | 1-12 | C 07 C 177/00<br>C 08 B 37/16<br>A 61 K 31/557 |
| Y | FR-A-2 258 372 (ARIES R.)<br>* Claims *<br>--- | 1,3 | |
| Y | FR-A-2 239 458 (ARIES R.)<br>* Claims *<br>----- | 1,3 | |
| | | | TECHNICAL FIELDS SEARCHED (Int. Cl.4) |
| | | | C 07 C 177/00 |

The present search report has been drawn up for all claims

| Place of search | Date of completion of the search | Examiner |
|---|---|---|
| THE HAGUE | 16-04-1985 | BERTE M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document